(19) Europäisches Patentamt / European Patent Office / Office européen des brevets

(11) EP 4 621 063 A1

(12) # EUROPEAN PATENT APPLICATION
published in accordance with Art. 153(4) EPC

(43) Date of publication:
**24.09.2025 Bulletin 2025/39**

(21) Application number: **23891589.6**

(22) Date of filing: **14.11.2023**

(51) International Patent Classification (IPC):
***C12P 21/08*** $^{(2006.01)}$ ***B01D 61/14*** $^{(2006.01)}$
***B01D 61/20*** $^{(2006.01)}$ ***B01D 63/02*** $^{(2006.01)}$
***B01D 69/00*** $^{(2006.01)}$ ***B01D 69/02*** $^{(2006.01)}$
***B01D 71/06*** $^{(2006.01)}$ ***B01D 71/34*** $^{(2006.01)}$
***B01D 71/68*** $^{(2006.01)}$ ***C12M 1/00*** $^{(2006.01)}$
***C12M 1/12*** $^{(2006.01)}$ ***C12P 1/00*** $^{(2006.01)}$
***C12P 21/02*** $^{(2006.01)}$ ***C12Q 1/02*** $^{(2006.01)}$
***C12Q 1/48*** $^{(2006.01)}$

(52) Cooperative Patent Classification (CPC):
**B01D 61/14; B01D 61/20; B01D 63/02; B01D 69/00; B01D 69/02; B01D 71/06; B01D 71/34; B01D 71/68; C07K 16/00; C12M 1/00; C12M 1/12; C12P 1/00; C12P 21/02; C12Q 1/02; C12Q 1/48**

(86) International application number:
**PCT/JP2023/040996**

(87) International publication number:
**WO 2024/106442 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.11.2022 JP 2022182807**
**15.11.2022 JP 2022182784**

(71) Applicant: **Asahi Kasei Life Science Corporation**
**Tokyo 100-0006 (JP)**

(72) Inventors:
- **MATSUDA, Shun**
  **Tokyo 100-0006 (JP)**
- **SUGIYAMA, Yuya**
  **Tokyo 100-0006 (JP)**
- **SAITO, Yuta**
  **Tokyo 100-0006 (JP)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **EVALUATION METHOD OF FILTRATION CONDITION AND PRODUCTION METHOD OF CELL PRODUCT**

(57) There is provided an evaluation method of a filtration condition, the evaluation method including, under a condition in which proliferation of cells is suppressed, feeding a cell broth containing cells from a culture vessel 11 containing the cell broth to a porous membrane 12, returning a cell broth that has passed through the porous membrane 12 without being filtered, to the culture vessel 11, and circulating the cell broth between the culture vessel 11 and the porous membrane 12; and evaluating one or more filtration conditions with the porous membrane 12.

EP 4 621 063 A1

Fig. 1

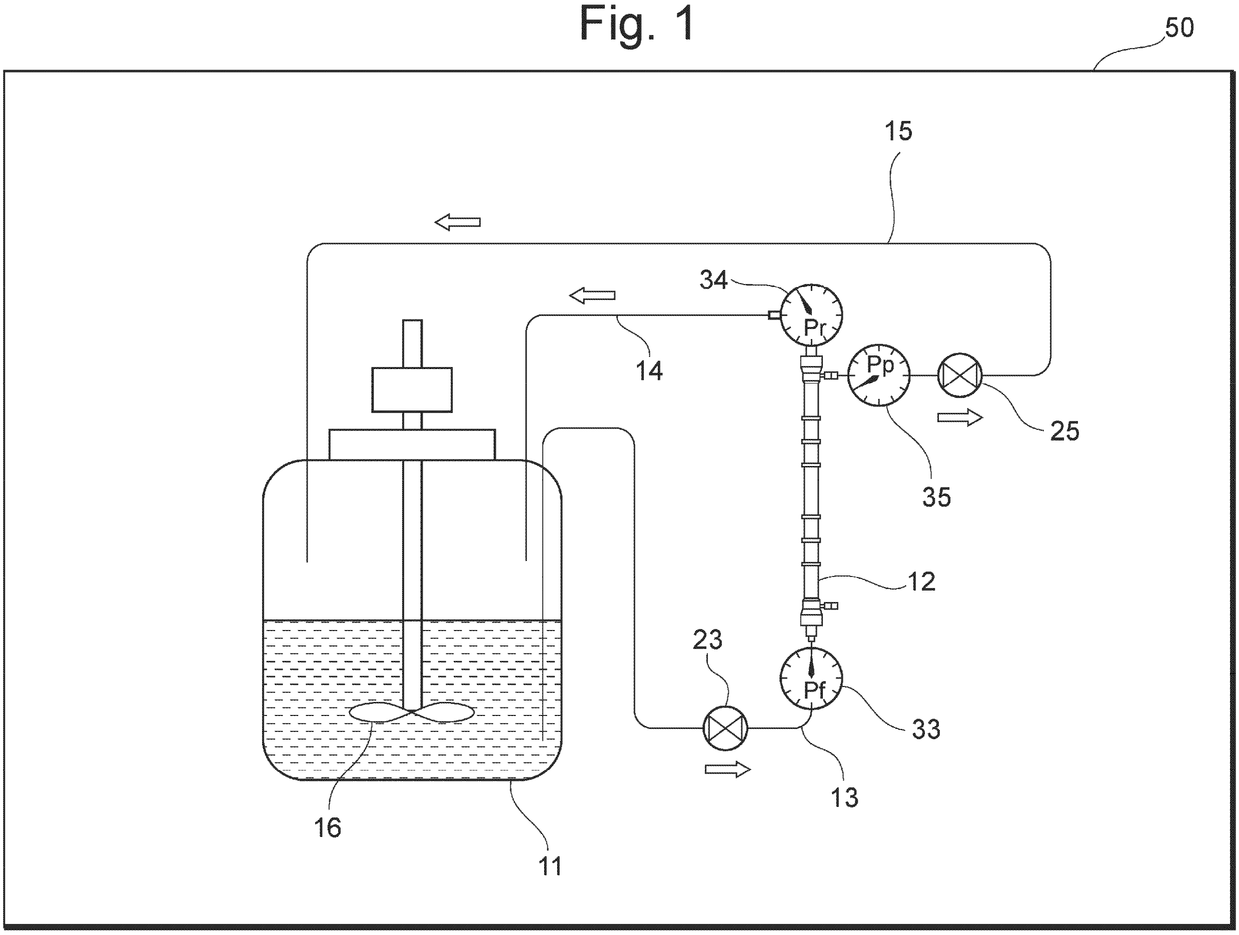

## Description

### Technical Field

The present invention relates to a filtration technique and specifically to an evaluation method of a filtration condition and a method for producing a product by cells.

### Background Art

A cell culture technique is an essential technique in the production of various biopharmaceutical products such as an antibody, a growth hormone, and insulin, and it has significantly contributed to recent advancements in medicine. Among the biopharmaceutical products, in particular, an antibody pharmaceutical drug is attracting attention. Producing monoclonal antibodies efficiently and stably by culturing antibody-producing cells is one of the industrially important themes.

The industrial cell culture methods aimed at producing useful products of cells such as antibodies are broadly classified into two methods of an adherent culture method and a suspension culture method (floating culture method). In the adherent culture method, cells adhere to an inner surface of a culture vessel. In the suspension culture method, cells float in a cell broth. Among these, the suspension culture method has become mainstream due to the ease of scale-up, the ease of control in terms of large scale, and the like.

A method where cells are cultured while filtering out an old cell broth containing a product by cells while supplying a fresh culture medium into a culture vessel at a constant rate, thereby discharging the old cell broth out of the culture vessel at a constant rate has been proposed in the suspension culture method in order to culture cells in large quantities and at a high density and to continuously produce a product by cells with high efficiency. This method of culture is generally referred to as continuous culture or perfusion culture (see, for example, Patent Literatures 1 to 5). In continuous culture, an amount of a culture medium supplied to a culture vessel can be controlled to be equal to an amount of a cell broth discharged from the culture vessel. In continuous culture, it is important to efficiently separate cells in a cell broth from an old cell broth and a product by the cells over a long period, thereby taking out the old cell broth and the product by the cells from the culture vessel and continuously maintaining the cell growth environment within the culture vessel under optimal conditions for a long period of time.

### Citation List

#### Patent Literatures

Patent Literature 1: Japanese Patent Application Laid-Open No. 2018-76291
Patent Literature 2: Japanese Patent Application Laid-Open No. 2009-45019
Patent Literature 3: Japanese Patent Application Laid-Open No. 2021-48776
Patent Literature 4: Japanese Patent No. 5696479
Patent Literature 5: PCT Japanese Translation Patent Publication No. 2022-516516

### Summary of Invention

#### Technical Problem

In continuous culture, a porous membrane is used to separate cells in a cell broth of a culture vessel from an old cell broth and a product by the cells. A method of appropriately evaluating a filtration condition with a porous membrane is desired. Therefore, an object of the present invention is to provide an evaluation method of a filtration condition, which can appropriately evaluate a filtration condition for a cell broth, and a method for producing a product by cells.

#### Solution to Problem

[1] According to an aspect of the present invention, there is provided an evaluation method of a filtration condition, the evaluation method including, under a condition in which proliferation of cells is suppressed, feeding a cell broth containing cells from a culture vessel containing the cell broth to a porous membrane, returning a cell broth that has passed through the porous membrane without being filtered, to the culture vessel, and circulating the cell broth between the culture vessel and the porous membrane; and evaluating one or more filtration conditions with the porous membrane.

[2] In the evaluation method of a filtration condition according to [1] described above, in the returning of the cell broth that has passed through the porous membrane without being filtered to the culture vessel, the cell broth that has passed through the porous membrane without being filtered and a cell broth that has been filtered through the porous membrane may be returned to the culture vessel.

[3] The evaluation method of a filtration condition according to [1] or [2] described above may further include:

evaluating filtration performance of the porous membrane under the one or more filtration conditions,
in which the one or more filtration conditions with the porous membrane are evaluated based on the filtration performance of the porous membrane.

[4] In the evaluation method of a filtration condition according to any of [1] to [3] described above, the condition in which the proliferation of cells is suppressed may be a condition in which cell cycle progression is suppressed.

[5] In the evaluation method of a filtration condition according to any of [1] to [4] described above, the condition in which the proliferation of cells is suppressed may be at least one of an atmospheric temperature or a cell broth temperature, which is 15°C or lower.

[6] In the evaluation method of a filtration condition according to any of [1] to [5] described above, the at least one of the atmospheric temperature or the cell broth temperature, which is 15°C or lower, may be at least one of an atmospheric temperature or a cell broth temperature, which is 10°C or lower or 5°C or lower.

[7] In the evaluation method of a filtration condition according to any of [1] to [6] described above, the condition in which the proliferation of cells is suppressed may be a condition in which an activity of an enzyme of the cells is suppressed.

[8] In the evaluation method of a filtration condition according to [7] described above, the enzyme may be a cyclin-dependent kinase.

[9] In the evaluation method of a filtration condition according to any of [1] to [8] described above, the condition in which the proliferation of cells is suppressed may be a presence of a cell cycle inhibitor in the cell broth.

[10] In the evaluation method of a filtration condition according to any of [1] to [9] described above, the one or more filtration conditions with the porous membrane may be evaluated based on a permeation rate of a product by the cells in the porous membrane.

[11] In the evaluation method of a filtration condition according to any of [1] to [10] described above, the one or more filtration conditions with the porous membrane may be evaluated based on a permeation flux of the porous membrane.

[12] In the evaluation method of a filtration condition according to any of [1] to [11] described above, the one or more filtration conditions with the porous membrane may be evaluated based on a transmembrane pressure of the porous membrane.

[13] In the evaluation method of a filtration condition according to any of [1] to [12] described above, the one or more filtration conditions with the porous membrane may be evaluated based on a turbidity of a filtrate.

[14] In the evaluation method of a filtration condition according to any of [1] to [13] described above, the one or more filtration conditions may be one condition or a plurality of conditions of a structure, a material, or physical properties of the porous membrane.

[15] In the evaluation method of a filtration condition according to any of [1] to [14] described above, the one or more filtration conditions may be one condition or a plurality of conditions of the cell broth.

[16] In the evaluation method of a filtration condition according to any of [1] to [15] described above, the one or more filtration conditions may be one condition or a plurality of conditions of a cell density in the cell broth.

[17] In the evaluation method of a filtration condition according to any of [1] to [16] described above, the one or more filtration conditions may be one condition or a plurality of conditions of a flow rate of the cell broth which is fed to the porous membrane.

[18] In the evaluation method of a filtration condition according to any of [1] to [17] described above, the one or more filtration conditions may be one condition or a plurality of conditions of a flow rate of the cell broth that has been filtered through the porous membrane.

[19] In the evaluation method of a filtration condition according to any of [1] to [18] described above, the one or more filtration conditions may be one condition or a plurality of conditions of a shear stress on a liquid contact surface of the porous membrane due to a flow of the cell broth which is fed to the porous membrane.

[20] In the evaluation method of a filtration condition according to any of [1] to [19] described above, a path including the culture vessel and the porous membrane through which the cell broth circulates may be disposed in a temperature management chamber.

[21] In the evaluation method of a filtration condition according to any of [1] to [20] described above, in circulating the cell broth between the culture vessel and the porous membrane, an active operation for maintaining a composition of the cell broth may not be performed.

[22] In the evaluation method of a filtration condition according to any of [1] to [21] described above, in circulating the cell broth between the culture vessel and the porous membrane, a culture medium may not be added from an outside to a path including the culture vessel and the porous membrane through which the cell broth circulates.

[23] In the evaluation method of a filtration condition according to any of [1] to [22] described above, in circulating the cell broth between the culture vessel and the porous membrane, at least one of dissolved oxygen or pH of the cell broth may not be controlled.

[24] In the evaluation method of a filtration condition according to any of [1] to [23] described above, in circulating the cell broth between the culture vessel and the porous membrane, the cells may not be bled from a path including the culture vessel and the porous membrane through which the cell broth circulates.

[25] In the evaluation method of a filtration condition according to any of [1] to [24] described above, in circulating the cell broth between the culture vessel and the porous membrane, the cell broth may be collected for sampling from a path including the culture vessel and the porous membrane through which the cell broth circulates.

[26] The evaluation method of a filtration condition according to [25] described above may further include measuring a cell density in the sampled cell broth.

[27] The evaluation method of a filtration condition according to [25] or [26] described above may further include measuring a viability of the cells in the sampled cell broth.

[28] The evaluation method of a filtration condition according to any of [25] to [27] described above may further include measuring a concentration of a product by the cells in the sampled cell broth.

[29] The evaluation method of a filtration condition according to any of [25] to [28] described above may further include measuring a turbidity in the sampled cell broth.

[30] In the evaluation method of a filtration condition according to any of [1] to [29] described above, the porous membrane may be a hollow fiber membrane.

[31] In the evaluation method of a filtration condition according to any of [1] to [30] described above, the porous membrane may be a microfiltration membrane.

[32] According to an aspect of the present invention, there is provided a method for producing a product by cells, the method including:

under a condition in which cells are proliferated, feeding a cell broth containing cells from a culture vessel containing the cell broth to a porous membrane using a filtration condition, returning a cell broth that has passed through the porous membrane without being filtered, to the culture vessel, recovering a cell broth containing a product by the cells, the cell broth having been filtered through the porous membrane, and circulating at least a part of the cell broth between the culture vessel and the porous membrane,

in which the filtration condition is a filtration condition obtained by, under a condition in which the proliferation of cells is suppressed, feeding the cell broth containing the cells from the culture vessel containing the cell broth to the porous membrane, returning a cell broth that has passed through the porous membrane without being filtered, to the culture vessel, circulating the cell broth between the culture vessel and the porous membrane, and evaluating one or more filtration conditions with the porous membrane.

[33] In the method for producing a product by cells according to [32] described above, in a case of evaluating the one or more filtration conditions with the porous membrane, in the returning of the cell broth that has passed through the porous membrane without being filtered to the culture vessel, the cell broth that has passed through the porous membrane without being filtered and a cell broth that has been filtered through the porous membrane may be returned to the culture vessel.

[34] In the method for producing a product by cells according to [32] or [33] described above, the condition in which the cells are proliferated may be a condition in which a cell cycle progresses.

[35] In the method for producing a product by cells according to any of [32] to [34] described above, the condition in which the cells are proliferated may be at least one of an atmospheric temperature or a cell broth temperature, which is 15°C or more.

[36] In the method for producing a product by cells according to [35] described above, the at least one of the atmospheric temperature or the cell broth temperature, which is higher than 15°C may be at least one of an atmospheric temperature or a cell broth temperature, which is higher than 20°C, 25°C, 30°C, or 35°C.

[37] In the method for producing a product by cells according to any of [32] to [36] described above, the condition in which the cells are proliferated may be a condition in which an enzyme of the cells is activated.

[38] In the method for producing a product by cells according to [37] described above, the enzyme may be a cyclin-dependent kinase.

[39] In the method for producing a product by cells according to any of [32] to [38] described above, the condition in which the cells are proliferated may be an absence of a cell cycle inhibitor in the cell broth.

[40] In the method for producing a product by cells according to any of [32] to [39] described above, the condition in which the proliferation of cells is suppressed may be a condition in which cell cycle progression is suppressed.

[41] In the method for producing a product by cells according to any of [32] to [40] described above, the condition in which the proliferation of cells is suppressed may be at least one of an atmospheric temperature or a cell broth temperature, which is 15°C or lower.

[42] In the method for producing a product by cells according to [41] described above, the at least one of the atmospheric temperature or the cell broth temperature, which is 15°C or lower, may be at least one of an atmospheric temperature or a cell broth temperature, which is 10°C or lower or 5°C or lower.

[43] In the method for producing a product by cells according to any of [32] to [42] described above, the condition in which the proliferation of cells is suppressed may be a condition in which an activity of an enzyme of the cells is suppressed.

[44] In the method for producing a product by cells according to [43] described above, the enzyme may be a cyclin-dependent kinase.

[45] In the method for producing a product by cells according to any of [32] to [44] described above, the condition in which the proliferation of cells is suppressed may be a presence of a cell cycle inhibitor in the cell broth.

[46] In the method for producing a product by cells according to any of [32] to [45] described above, the one or more filtration conditions with the porous membrane may have been evaluated based on a permeation rate of the product by the cells in the porous membrane.

[47] In the method for producing a product by cells according to any of [32] to [46] described above, the one or more filtration conditions with the porous membrane may have been evaluated based on a permeation flux of the porous membrane.

[48] In the method for producing a product by cells according to any of [32] to [47] described above, the one or more filtration conditions with the porous membrane may have been evaluated based on a transmembrane pressure of the porous membrane.

[49] In the method for producing a product by cells according to any of [32] to [48] described above, the one or more filtration conditions with the porous membrane may have been evaluated based on a turbidity of a filtrate.

[50] In the method for producing a product by cells according to any of [32] to [49] described above, the one or more filtration conditions may be one condition or a plurality of conditions of a structure, a material, or physical properties of the porous membrane.

[51] In the method for producing a product by cells according to any of [32] to [50] described above, the one or more filtration conditions may be one condition or a plurality of conditions of the cell broth.

[52] In the method for producing a product by cells according to any of [32] to [51] described above, the one or more filtration conditions may be one condition or a plurality of conditions of a cell density in the cell broth.

[53] In the method for producing a product by cells according to any of [32] to [52] described above, the one or more filtration conditions may be one condition or a plurality of conditions of a flow rate of the cell broth which is fed to the porous membrane.

[54] In the method for producing a product by cells according to any of [32] to [53] described above, the one or more filtration conditions may be one condition or a plurality of conditions of a flow rate of the cell broth that has been filtered through the porous membrane.

[55] In the method for producing a product by cells according to any of [32] to [54] described above, the one or more filtration conditions may be one condition or a plurality of conditions of a shear stress on a liquid contact surface of the porous membrane due to a flow of the cell broth which is fed to the porous membrane.

[56] In the method for producing a product by cells according to any of [32] to [55] described above, in a case of evaluating the one or more filtration conditions with the porous membrane, a path including the culture vessel and the porous membrane through which the cell broth circulates may be disposed in a temperature management chamber.

[57] In the method for producing a product by cells according to any of [32] to [56] described above, in a case of evaluating the plurality of filtration conditions with the porous membrane, in circulating the cell broth between the culture vessel and the porous membrane, an active operation for maintaining a composition of the cell broth may not be performed.

[58] In the method for producing a product by cells according to any of [32] to [57] described above, in a case of evaluating the plurality of filtration conditions with the porous membrane, in circulating the cell broth between the culture vessel and the porous membrane, a culture medium may not be added from an outside to a path including the culture vessel and the porous membrane through which the cell broth circulates.

[59] In the method for producing a product by cells according to any of [32] to [58] described above, in a case of evaluating the plurality of filtration conditions with the porous membrane, in circulating the cell broth between the culture vessel and the porous membrane, at least one of dissolved oxygen or pH of the cell broth may not be controlled.

[60] In the method for producing a product by cells according to any of [32] to [59] described above, in a case of evaluating the plurality of filtration conditions with the porous membrane, in circulating the cell broth between the culture vessel and the porous membrane, the cells may not be bled from a path including the culture vessel and the porous membrane through which the cell broth circulates.

[61] In the method for producing a product by cells according to any of [32] to [60] described above, in a case of evaluating the one or more filtration conditions with the porous membrane, in circulating the cell broth between the culture vessel and the porous membrane, the cell broth may be collected for sampling from a path including the culture vessel and the porous membrane through which the cell broth circulates.

[62] The method for producing a product by cells according to [61] described above may further include measuring a cell density in the sampled cell broth.

[63] The method for producing a product by cells according to [61] or [62] described above may further include measuring a viability of the cells in the sampled cell broth.

[64] The method for producing a product by cells according to any of [61] to [63] described above may further include measuring a concentration of the product by the cells in the sampled cell broth.

[65] The method for producing a product by cells according to any of [61] to [64] described above may further include measuring a turbidity in the sampled cell broth.

[66] In the method for producing a product by cells according to any of [32] to [65] described above, the porous membrane may be a hollow fiber membrane.

[67] In the method for producing a product by cells according to any of [32] to [66] described above, the porous membrane may be a microfiltration membrane.

[68] In the method for producing a product by cells according to any of [32] to [67] described above, the cells may be subjected to perfusion culture.

Advantageous Effect of Invention

According to the present invention, it is possible to provide an evaluation method of a filtration condition, which can appropriately evaluate a filtration condition for a cell broth, and a method for producing a product by cells.

Brief Description of Drawings

[Figure 1] Figure 1 is a schematic view illustrating an evaluation system of a filtration condition for a hollow fiber membrane according to an embodiment.

[Figure 2] Figure 2 is a schematic view illustrating a system for producing a product by cells according to an embodiment.

[Figure 3] Figure 3 is a graph showing a temporal change of a cell density in a cell broth that circulates between a culture vessel and a hollow fiber membrane according to Examples.

[Figure 4] Figure 4 is a graph showing a temporal change of a cell density in a cell broth that circulates between a culture vessel and a hollow fiber membrane according to Examples.

[Figure 5] Figure 5 is a graph showing a temporal change of a viability of cells in a cell broth that circulates between a culture vessel and a hollow fiber membrane according to Examples.

[Figure 6] Figure 6 is a graph showing a temporal change of a viability of cells in a cell broth that circulates between a culture vessel and a hollow fiber membrane according to Examples.

[Figure 7] Figure 7 is a graph showing a temporal change of a concentration of an antibody in a cell broth that circulates between a culture vessel and a hollow fiber membrane according to Examples.

[Figure 8] Figure 8 is a graph showing a temporal change of a concentration of an antibody in a cell broth that circulates between a culture vessel and a hollow fiber membrane according to Examples.

[Figure 9] Figure 9 is a graph showing a temporal change of a cell density in a cell broth that circulates between a culture vessel and a hollow fiber membrane according to Comparative Examples.

[Figure 10] Figure 10 is a graph showing a temporal change of a viability of cells in a cell broth that circulates between a culture vessel and a hollow fiber membrane according to Comparative Examples.

[Figure 11] Figure 11 is a graph showing a temporal change of a concentration of an antibody in a cell broth that circulates between a culture vessel and a hollow fiber membrane according to Comparative Examples.

[Figure 12] Figure 12 is a graph showing a temporal change of a cell density in a cell broth in a culture vessel according to Reference Examples.

[Figure 13] Figure 13 is a graph showing a temporal change of a viability of cells in a cell broth in a culture vessel according to Reference Examples.

[Figure 14] Figure 14 is a graph showing a temporal change of a concentration of an antibody in a cell broth in a culture vessel according to Reference Examples.

[Figure 15] Figure 15 is a graph showing a temporal change of a cell density in a cell broth in a culture vessel according to Reference Examples.

[Figure 16] Figure 16 is a graph showing a temporal change of a viability of cells in a cell broth in a culture vessel according to Reference Examples.

[Figure 17] Figure 17 is a graph showing a temporal change of a concentration of an antibody in a cell broth in a culture vessel according to Reference Examples.

[Figure 18] Figure 18 is a graph showing a temporal change of a cell density in a cell broth in a culture vessel according to Reference Examples.

[Figure 19] Figure 19 is a graph showing a temporal change of a viability of cells in a cell broth in a culture vessel according to Reference Examples.

[Figure 20] Figure 20 is a graph showing a temporal change of a concentration of an antibody in a cell broth in a culture vessel according to Reference Examples.

Description of Embodiments

Hereinafter, embodiments (hereinafter, referred to as "the present embodiments") for carrying out the present invention will be described in detail. It is noted that the present embodiment is provided to facilitate understanding of the present invention and thus is not intended to limit the present invention. The present invention is not limited to the present embodiment and thus can be implemented with various modifications within the scope of the present invention.

With reference to Figure 1, an evaluation method of a filtration condition according to the present embodiment includes under a condition in which proliferation of cells is suppressed, feeding a cell broth containing cells from a culture vessel 11 containing the cell broth to a porous membrane 12, returning a cell broth that been passed through the porous membrane 12 without being filtered, to the culture vessel 11, and circulating the cell broth between the culture vessel 11 and the porous membrane 12; evaluating filtration performance of the porous membrane 12 under the one or more filtration conditions; and evaluating one or more filtration conditions with the porous membrane 12 based on the filtration performance of the porous membrane 12. In the returning of the cell broth that has passed through the porous membrane 12 without being filtered therethrough to the culture vessel 11, the cell broth that has passed through the porous membrane 12 without being filtered therethrough and a cell broth that has been filtered through the porous membrane 12 may be returned to the culture vessel 11. It should be noted that the recovery of the cell broth without returning the entire amount of the cell broth to the culture vessel 11 is not hindered as long as a culture medium having the same amount as the recovered cell broth is supplemented in the cell broth in the culture vessel 11. The cell broth may be recovered by recovering a cell broth filtered through the porous membrane 12 without returning a part or whole of the filtered cell broth to the culture vessel 11. The amount of the cell broth to be recovered is not limited, and the variation of the cell density and the concentration of the product contained in the cell broth in the culture vessel 11 is suppressed by supplementing the system with the same amount of the culture medium. It is noted that the recovery is distinguished from sampling described later, which is carried out for the purpose of analyzing the cell broth.

The culture vessel 11 is, for example, a container having an internal space closed from the outside air. However, the culture vessel 11 may be equipped with a ventilation port or a filter for keeping the internal pressure constant. The ventilation port or the filter preferably has a pore diameter of 0.2 μm or less in order to prevent external bacteria from entering the inside of the culture vessel 11. The culture vessel 11 may be equipped with a stirring device 16 for stirring the cell broth in the culture vessel 11. Cells are subjected to floating culture in the cell broth in the culture vessel 11.

The cells to be cultured in the culture vessel 11 are not particularly limited. The cell may be derived from an animal including a human or may be derived from a microorganism. The cell may be a eukaryotic cell or may be a prokaryotic cell. Examples of the animal include mammals, reptiles, birds, amphibians, fish, and insects. The cell may be a genetically recombinant cell. Examples of the cells include a Chinese hamster ovary (CHO) cell, an HEK cell, a BHK-21 cell, an Sp2/0 cell, an SP2/0-Ag14 cell, an NS0 cell, a Vero cell, a PER.C6 cell, a yeast, Bacillus subtilis, and Escherichia coli.

The cells produce, for example, a product that can be used as a pharmaceutical drug and release the product into the cell broth. Examples of the product that can be used as a pharmaceutical drug include a peptide, a protein, and a virus (including a virus-like particle). Examples of the protein include an antibody, a hormone, a cytokine, a growth factor, an enzyme, and a blood plasma protein. The protein may be a recombinant protein.

The antibody may be a monoclonal antibody or may be a polyclonal antibody. The antibody may be a human antibody or may be an antibody protein derived from mammals other than a human, such as a bovine and mouse. Alternatively, the antibody may be a chimeric antibody protein with human IgG or a humanized antibody. The chimeric antibody with human IgG is antibody in which although variable regions are derived from an organism other than a human, such as a mouse, constant regions other than the variable regions are substituted those of human-derived immunoglobulin. In addition, the humanized antibody is an antibody in which although a complementarity-determining region (CDR) in the variable region is derived from an organism other than a human, a framework region (FR) other than the complementarity-determining region is derived from a human. The humanized antibody has reduced immunogenicity as compared with the chimeric antibody.

The shape of the porous membrane 12 is not particularly limited. Examples of the porous membrane include a hollow fiber membrane, a flat membrane, and a tubular membrane. In the following description, an example in which the porous membrane 12 is a hollow fiber membrane will be described. In the porous membrane 12, a surface to which a cell broth is supplied is referred to as a primary side of the hollow fiber membrane. In addition, a surface on which a permeated solution permeated through the hollow fiber membrane flows out is referred to as a secondary side of the hollow fiber membrane. In an embodiment in which a cell broth to be filtered is supplied to an inner peripheral surface, the inner peripheral surface of the hollow fiber membrane serves as the primary side, and the outer peripheral surface of the hollow fiber membrane serves as the secondary side. In an embodiment in which a cell broth to be filtered is supplied to an outer peripheral surface, the outer peripheral surface of the hollow fiber membrane serves as the primary side, and the inner peripheral surface of the hollow fiber membrane serves as the secondary side.

The type of the porous membrane 12 is not particularly limited. Examples of the porous membrane include a coarse filtration membrane, a microfiltration membrane, an ultrafiltration membrane, a dialysis membrane, a nanofiltration membrane, a reverse osmosis membrane, and an osmosis membrane.

The filtration method with the porous membrane 12 may be a tangential flow filtration (TFF) method. The tangential flow filtration method is a filtration method in which a cell broth is allowed to flow in a direction parallel to the primary side surface of the hollow fiber membrane on the primary side surface of the hollow fiber membrane. The tangential flow filtration method includes an alternating tangential flow filtration (ATF) method. In the present embodiment, in a case of simply being referred to as a tangential flow filtration (TFF) method, it may refer to a filtration method in which a cell broth is allowed to flow in one direction on the primary side surface of the hollow fiber membrane. The alternating tangential flow filtration (ATF) method refers to a filtration method in which a cell broth is allowed to flow so that it flows back and forth on the primary side surface of the hollow fiber membrane.

In the example illustrated in Figure 1, a flow channel 13 for feeding a cell broth in the culture vessel 11 to the porous membrane 12, a flow channel 14 for returning a cell broth that has passed through the porous membrane 12 without being filtered through the porous membrane 12, to the culture vessel 11, and optionally, a flow channel 15 for returning a cell broth that has been filtered through the porous membrane 12, to the culture vessel 11, are disposed between the culture vessel 11 and the porous membrane 12. The cell broth flowing through the flow channel 13 may contain cells and a product by the cells. The cell broth flowing through the flow channel 14, which has passed through the porous membrane 12 without being filtered therethrough, may contain cells and a product by the cells. The cell broth flowing through the flow channel 15, which has been filtered through the porous membrane 12, may contain a product by cells.

For example, the flow channel 13 is equipped with a pump 23 for feeding the cell broth in the culture vessel 11 to the porous membrane 12. Examples of the pump include a diaphragm pump, a tube pump, a centrifugal pump, and a rotary pump; however, examples thereof are not limited thereto. The flow channel 13 may be equipped with a pressure gauge 33 that measures the pressure of the cell broth that is supplied to the porous membrane 12. The flow channel 13 may be equipped with a flowmeter that measures at least one of a flow speed or a flow rate of the cell broth that flows in the flow channel 13. The flow channel 13 may be equipped with a thermometer that measures the temperature of the cell broth that flows in the flow channel 13. The flow channel 13 may be equipped with a sampling unit for sampling the cell broth that flows in the flow channel 13. The sampling unit is closed except for the time of sampling.

The flow channel 14 may be equipped with a pump for feeding, to the culture vessel 11, a cell broth that has passed through the hollow portion without passing through the micropore portion of the porous membrane 12 and has not been filtered through the porous membrane 12. It is noted that the pump may be provided in both the flow channel 13 and the flow channel 14 or may be provided in any one of the flow channel 13 or the flow channel 14. The flow channel 14 may be equipped with a pressure gauge 34 that measures the pressure of the cell broth that has passed through the porous membrane 12. The flow channel 14 may be equipped with a flowmeter that measures at least one of a flow speed or a flow rate of the cell broth that flows in the flow channel 14. The flow channel 14 may be equipped with a thermometer that measures the temperature of the cell broth that flows in the flow channel 14. The flow channel 14 may be equipped with a sampling unit for sampling the cell broth that flows in the flow channel 14. The sampling unit is closed except for the time of sampling.

The flow channel 15 is equipped with, for example, a pump 25 for feeding the cell broth that has passed through the micropore portion of the porous membrane 12 and has been filtered through the porous membrane 12, to the culture vessel 11. The flow channel 15 may be equipped with a pressure gauge 35 that measures the pressure of the cell broth that has been filtered through the porous membrane 12. The flow channel 15 may be equipped with a flowmeter that measures at least one of a flow speed or a flow rate of the cell broth that flows in the flow channel 15. The flow channel 15 may be equipped with a sampling unit for sampling the cell broth that flows in the flow channel 15. The sampling unit is closed except for the time of sampling.

The culture vessel 11 and the porous membrane 12 form at least a part of a path through which the cell broth circulates. In addition, the flow channels 13, 14, and 15 form at least a part of a path through which the cell broth circulates. The path through which the cell broth circulates may be closed from the outside; however, as described above, the culture vessel 11 may be equipped with a ventilation port or a filter for keeping the internal pressure constant.

The culture vessel 11 and the porous membrane 12 may be disposed in a temperature management chamber 50 that manages the internal temperature. The flow channels 13, 14, and 15 may be disposed in the temperature management chamber 50. The temperature management chamber 50 manages the atmospheric temperatures of the culture vessel 11, the porous membrane 12, and the flow channels 13, 14, and 15. The temperature management chamber 50 may be a thermostatic chamber or a refrigerator, or it may be a booth, a room, or a building, which is maintained at a constant temperature.

The condition in which the proliferation of cells is suppressed is not particularly limited; however, it is, for example, a condition in which the cell cycle progression is suppressed. The condition in which the cell cycle progression is suppressed is, for example, a low atmospheric temperature of 15°C or lower of the culture vessel 11 and the porous membrane 12 or a temperature of 15°C or lower of the cell broth. The atmospheric temperature or the temperature of the cell broth is preferably 10°C or lower or 5°C or lower. It is noted that from the viewpoint of suppressing the death of cells, the atmospheric temperatures of the culture vessel 11 and the porous membrane 12 or the temperature of the cell broth is preferably 0°C or higher, 1°C or higher, 2°C or higher, or 3°C or higher. The temperature of the cell broth may be directly measured, or the temperature of the cell broth may be measured by estimating it from the atmospheric temperatures of the culture vessel 11 and the porous membrane 12. The temperature of the cell broth may be directly controlled by a temperature control device based on the measured temperature of the cell broth, or the atmospheric temperatures of the culture vessel 11 and the porous membrane 12 may be controlled by a temperature control device. The condition in which the cell cycle progression is suppressed may **be,** for example, a condition in which an enzymatic activity of a cyclin-dependent kinase or the like in cells is suppressed. The condition in which the cell cycle progression is suppressed may be the presence of a cell cycle inhibitor in the cell broth. Under a condition in which the proliferation of cells is suppressed, the activity of cells is decreased, the cell cycle progression is suppressed, whereby the division of cells is suppressed, and the production of the product is also suppressed.

In the present embodiment, both the cell broth that has passed through the porous membrane 12 without being filtered therethrough and the cell broth that has been filtered through the porous membrane 12 may be returned to the culture vessel 11. In this case, under the condition in which the proliferation of cells is suppressed, the total amount of cells and the total amount of the products in the system are maintained, and thus the variation of the cell density and the concentration of the product contained in the cell broth that circulates between the culture vessel 11 and the porous membrane 12 is suppressed.

Under the condition in which the proliferation of cells is suppressed, the activity of cells is decreased, and the amount of cell broth components required by cells is also decreased. Therefore, it is not necessary to add a culture medium from the outside to the path through which the cell broth circulates. In addition, the variation of the cell density and the concentration of the product contained in the cell broth is suppressed by not adding a culture medium. Further, under the condition in which the proliferation of cells is suppressed, the activity of cells is decreased, and the variation of the dissolved oxygen concentration (DO) and the pH in the cell broth is suppressed. Therefore, it is not necessary to control the dissolved oxygen concentration and the pH by adding a gas such as oxygen or carbon dioxide from the outside to the path through which the cell broth circulates. Furthermore, under the condition in which the proliferation of cells is suppressed, the cell density in the cell broth is substantially constant, and thus the cells do not have to be bled to the outside from the path through which the cell broth circulates. Therefore, according to the evaluation method of a filtration condition according to the present embodiment, it is possible to simplify the operation for cell culture. However, for example, a part of the cell broth may be sampled in order to monitor the cell density, the viability of the cells, and the concentration of the product by the cells. The sampling is distinguished from bleeding since the sampling is not intended to adjust the cell density.

One or more filtration conditions with the porous membrane 12 are evaluated based on the filtration performance of the porous membrane 12. The filtration performance of the porous membrane 12 may be indicated by, for example, at least one of the height of the permeation rate of the product by the cells in the porous membrane 12, the height of the shielding rate of the impurities of the porous membrane 12, the lowness of the transmembrane pressure of the porous membrane 12, the quantity of the amount of liquid that is capable of being filtered until the transmembrane pressure of the porous membrane 12 increases, the quantity of the amount of liquid that is capable of being filtered until the permeation flux of the porous membrane 12 starts to decrease, the lowness of the pressure loss in the porous membrane 12, the magnitude of the flow rate in the porous membrane 12, the speed of the flow speed in the porous membrane 12, and the speed of the permeation flux of the porous membrane 12.

One or more filtration conditions with the porous membrane 12 are evaluated based on whether or not the filtration performance of the porous membrane 12 satisfies a predetermined standard or is relatively excellent, depending on one or more filtration conditions with the porous membrane 12. Alternatively, at least a part of the filtration conditions may be extracted among the plurality of filtration conditions with the porous membrane 12 based on whether or not the filtration performance of the porous membrane 12 satisfies a predetermined standard or is relatively excellent.

In a case where the variation of the cell density and the concentration of the product contained in the cell broth supplied to the porous membrane 12 varies, it may be difficult to accurately evaluate the filtration performance of the porous membrane 12. On the other hand, as described above, in the present embodiment, the proliferation of cells is suppressed, and the variation of the cell density and the concentration of the product contained in the cell broth is suppressed. In a case where the cell broth filtered through the porous membrane 12 is returned to the culture vessel 11, the variation of the cell density and the concentration of the product contained in the cell broth is further suppressed. Therefore, according to the present embodiment, it is possible to evaluate the filtration performance of the porous membrane 12 while keeping the cell density and the concentration of the product contained in the cell broth substantially constant. In addition, in a case where the cell broth filtered through the porous membrane 12 is returned to the culture vessel 11, it may be possible to omit the replenishment of the culture medium to the culture vessel 11.

The one or more filtration conditions are, for example, one condition or a plurality of conditions of the structure of the porous membrane 12. The conditions of the structure of the porous membrane 12 are, for example, a membrane thickness, an inner diameter, an outer diameter, an opening ratio of a membrane surface, a void ratio, a pore diameter, an average pore diameter, variation of a pore diameter, a blocking pore diameter, a ratio of a pore diameter to a fiber diameter, anisotropy of a membrane surface, anisotropy of a membrane pore, a membrane surface roughness, and a change in pore diameter from a primary side to a secondary side. For example, a plurality of porous membranes 12 having various structures is prepared, and the filtration performance of each porous membrane 12 is evaluated. Further, it is evaluated whether or not one condition or a plurality of conditions of the structure of the porous membrane 12 gives filtration performance satisfying a predetermined standard or is relatively excellent.

The one or more filtration conditions are, for example, one condition or a plurality of conditions of the material of the porous membrane 12. For example, a plurality of porous membranes 12 made of various materials is prepared, and the filtration performance of each porous membrane 12 is evaluated. Further, it is evaluated whether or not one condition or a plurality of conditions of the material of the porous membrane 12 gives filtration performance satisfying a predetermined standard or is relatively excellent.

The one or more filtration conditions are, for example, one condition or a plurality of conditions of the physical properties of the porous membrane 12. The conditions of the physical properties of the porous membrane 12 are, for example, hydrophilicity, hydrophobicity, cationicity, anionicity, elastic limit pressure, and a bubble point. For example, a plurality of porous membranes 12 having various physical properties is prepared, and the filtration performance of each porous membrane 12 is evaluated. Further, it is evaluated whether or not one condition or a plurality of conditions of the physical properties of the porous membrane 12 gives filtration performance satisfying a predetermined standard or is relatively excellent.

The one or more filtration conditions are, for example, one condition or a plurality of conditions of the cell broth. The conditions of the cell broth are, for example, a contained substance of a cell broth, a concentration of each contained substance in a cell broth, a viscosity of a cell broth, a pH of a cell broth, an electrical conductivity of a cell broth, and a turbidity of a cell broth. Examples of the contained substance of the cell broth include a component of the cell broth, an anti-foaming agent, a salt, a nucleic acid such as DNA or RNA, a host cell-derived protein (HCP), a lipid, and polysaccharides. For example, a plurality of cell broths having different conditions is prepared, and the filtration performance of the porous membrane 12 in a case where each cell broth is used is evaluated. Further, it is evaluated whether or not one condition or a plurality of conditions of the cell broth gives filtration performance satisfying a predetermined standard or is relatively excellent.

The one or more filtration conditions are, for example, a plurality of conditions of a cell density in the cell broth. For example, a plurality of cell broths having different cell densities are prepared, and the filtration performance of the porous membrane 12 in a case where each cell broth is used is evaluated. Further, it is evaluated whether or not one condition or a plurality of conditions of the cell density gives filtration performance satisfying a predetermined standard or is relatively excellent.

The one or more filtration conditions are, for example, one condition or a plurality of conditions of a flow rate of the cell broth which is fed to the porous membrane 12. For example, the cell broth is fed to the porous membrane 12 at flow rates different each other, and the filtration performance of the porous membrane 12 in a case where each flow rate is used is evaluated. Further, it is evaluated whether or not one condition or a plurality of conditions of the flow rate of the cell broth fed to the porous membrane 12 gives filtration performance satisfying a predetermined standard or is relatively excellent. The flow rate may be indicated as a volumetric flow rate, may be indicated as a mass flow rate, may be indicated as a linear velocity, or may be indicated as a shear rate on the surface of the porous membrane 12.

The one or more filtration conditions are, for example, one condition or a plurality of conditions of a flow rate of the cell broth that has been filtered through the porous membrane 12. For example, the cell broth filtered through the porous membrane 12 is fed at flow rates different each other, and the filtration performance of the porous membrane 12 in a case where each flow rate is used is evaluated. Further, it is evaluated whether or not one condition or a plurality of conditions of the flow rate of the cell broth that has been filtered through the porous membrane 12 gives filtration performance satisfying a predetermined standard or is relatively excellent.

The one or more filtration conditions are, for example, one condition or a plurality of conditions of the shear stress on the liquid contact surface of the porous membrane 12. The liquid contact surface is a surface of the porous membrane 12, with which the liquid to be filtered comes into contact. In a case where the porous membrane 12 is a hollow fiber membrane, the surface thereof is a surface on the primary side. For example, the shear stress on the liquid contact surface of the porous membrane 12 is changed, and the filtration performance of the porous membrane 12 in a case where each shear stress is used is evaluated. Further, it is evaluated whether or not one condition or a plurality of conditions of the shear stress on the liquid contact surface of the porous membrane 12 gives filtration performance satisfying a predetermined standard or is relatively excellent.

According to the evaluation method of a filtration condition according to the present embodiment, while suppressing the variation of the cell density and the concentration of the product contained in the cell broth that circulates between the culture vessel 11 and the porous membrane 12, it is possible to select one or more filtration conditions under which the filtration performance of the porous membrane 12 satisfying a predetermined standard is allowed to be exhibited.

Next, with reference to Figure 2, a method for producing a product by cells according to the present embodiment includes under a condition in which cells are proliferated, feeding a cell broth containing cells from a culture vessel 111 containing the cell broth to a porous membrane 112 using a filtration condition obtained by the above-described evaluation method of a filtration condition, returning, to the culture vessel 111, a cell broth that has passed through the porous membrane 112 without being filtered therethrough, recovering a cell broth containing a product by the cells, the cell broth having been filtered through the porous membrane 112, and circulating at least a part of the cell broth between the culture vessel 111 and the porous membrane 112.

The conditions under which the cells are proliferated are not particularly limited as long as the cells produce a product and the product is continuously supplied into the cell broth; however, the conditions are for example, conditions under which the cell cycle progresses. The condition in which the cell cycle progresses is, for example, an atmospheric temperature of 15°C or higher of the culture vessel 111 and the porous membrane 112 or a temperature of 15°C or higher of the cell broth. The atmospheric temperature or the temperature of the cell broth is preferably a temperature higher than 20°C, 25°C, 30°C, or 35°C. In addition, from the viewpoint of suppressing the death of cells, the atmospheric temperature of the culture vessel 111 and the porous membrane 112 or the temperature of the cell broth is preferably 60°C or lower, 50°C or lower, or 40°C or lower. The temperature of the cell broth may be directly measured, or the temperature of the cell broth may be measured by estimating it from the atmospheric temperatures of the culture vessel 111 and the porous membrane 112. The temperature of the cell broth may be directly controlled by a temperature control device based on the measured temperature of the cell broth, or the atmospheric temperatures of the culture vessel 111 and the porous membrane 112 may be controlled by a temperature control device. The condition in which the cell cycle progresses may be, for example, a condition in which an enzyme such as a cyclin-dependent kinase in cells is activated. The condition in which the cell cycle progresses may be an absence of a cell cycle inhibitor in the cell broth.

It is preferable that the cells to be cultured in the culture vessel 111 are the same as the cells used in the evaluation method of a filtration condition. However, the cells maybe different from the cells used in the evaluation method of a filtration condition. In addition, it is preferable that the product by the cells which is filtered through the porous membrane 112 is the same as the product by the cells which has been filtered by the evaluation method of a filtration condition. However, the product may be different from the product by the cells which has been filtered by the evaluation method of a filtration condition.

A flow channel 113 for feeding a cell broth in the culture vessel 111 to the porous membrane 112 and a flow channel 114 for returning a cell broth that has passed through the porous membrane 112 without being filtered through the porous membrane 112, to the culture vessel 111, are disposed between the culture vessel 111 and the porous membrane 112. The cell broth flowing through the flow channel 113 may contain cells and a product by the cells. A cell broth flowing through the flow channel 114, which has passed through the hollow portion without passing through the micropore portion of the porous membrane 112 and has not been filtered through the porous membrane 112, may contain cells and a product by the cells. In addition, a flow channel 115 for recovering a cell broth filtered through the porous membrane 112 is connected to the porous membrane 112. A cell broth flowing through the flow channel 115, which has passed through the micropore portion of the porous membrane 112 and then has been filtered through the porous membrane 112 may contain a product by cells. The cell broth filtered through the porous membrane 112 is recovered in, for example, a container 201. The container 201 may be aseptically connected to the flow channel 115. The flow channel 115 through which the cell broth filtered through the porous membrane 112 flows may be directly connected to, for example, a column that is used for the next purification step. The cell broth filtered through the porous membrane 112 is not returned to the culture vessel 111.

For example, the flow channel 113 is equipped with a pump 123 for feeding the cell broth in the culture vessel 111 to the porous membrane 112. The flow channel 113 may be equipped with a pressure gauge 133 that measures the pressure of the cell broth that is supplied to the porous membrane 112. The flow channel 113 may be equipped with a flowmeter that measures at least one of a flow speed or a flow rate of the cell broth that flows in the flow channel 113. The flow channel 113 may be equipped with a thermometer that measures the temperature of the cell broth that flows in the flow channel 113. The flow channel 113 may be equipped with a sampling unit for sampling the cell broth that flows in the flow channel 113. The sampling unit is closed except for the time of sampling.

The flow channel 114 may be equipped with a pump for feeding, to the culture vessel 111, a cell broth that has passed through the hollow portion without passing through the micropore portion of the porous membrane 112 and has not been filtered through the porous membrane 112. It is noted that the pump may be provided in both the flow channel 113 and the flow channel 114 or may be provided in any one of the flow channel 113 or the flow channel 114. The flow channel 114 may be equipped with a pressure gauge 134 that measures the pressure of the cell broth that has passed through the porous membrane 112. The flow channel 114 may be equipped with a flowmeter that measures at least one of a flow speed or a flow rate of the cell broth that flows in the flow channel 114. The flow channel 114 may be equipped with a thermometer that measures the temperature of the cell broth that flows in the flow channel 114. The flow channel 114 may be equipped with a sampling unit for sampling the cell broth that flows in the flow channel 114. The sampling unit is closed except for the time of sampling.

The flow channel 115 is equipped with, for example, a pump 125 for feeding the cell broth filtered through the porous membrane 112. The flow channel 115 may be equipped with a pressure gauge 135 that measures the pressure of the cell broth that has been filtered through the porous membrane 112. The flow channel 115 may be equipped with a flowmeter that measures at least one of a flow speed or a flow rate of the cell broth that flows in the flow channel 115. The flow channel 115 may be equipped with a sampling unit for sampling the cell broth that flows in the flow channel 115. The sampling unit is closed except for the time of sampling.

The culture vessel 111 and the porous membrane 112 form at least a part of a path through which the cell broth circulates. In addition, the flow channels 113 and 114 form at least a part of a path through which the cell broth circulates.

A flow channel 116 for supplying a culture medium to the culture vessel 111 may be connected to the culture vessel 111. The flow channel 116 is connected to, for example, a culture medium reservoir 216 that contains a culture medium. For example, the flow channel 116 is equipped with a pump 126 for feeding a culture medium to the culture vessel 111. For example, the pump 125 and the pump 126 are controlled such that the amount of the cell broth that is filtered through the porous membrane 112 but is not returned to the culture vessel 111 and the amount of the culture medium that is supplied to the culture vessel 111 are the same. This control may be carried out by observing the liquid level height using a liquid level sensor (level sensor) installed in the culture vessel 111 so that the liquid level height is constant, or it may be carried out by measuring the weight of the entire culture vessel 111 containing the cell broth so that the weight is constant.

A flow channel 117 for supplying air containing carbon dioxide to the culture vessel 111 may be connected to the culture vessel 111. The flow channel 117 is connected to, for example, a container 217 that contains air containing carbon dioxide. In addition, a flow channel 118 for supplying oxygen to the culture vessel 111 may be connected to the culture vessel 111. The flow channel 118 is connected to, for example, a container 218 that contains oxygen.

A flow channel 119 for discharging at least a part of the cells in the culture vessel 111 may be connected to the culture vessel 111. For example, by discharging at least a part of the cells in the culture vessel 111 using the flow channel 119, the cell density in the cell broth in the culture vessel 111 is kept constant, which suppresses a shortage of oxygen and cell broth components in the cell broth due to an increase in the cell density, or an increase in the concentration of impurities. Discharging at least a part of the cells in the culture vessel 111 is referred to as bleeding.

The culture vessel 111 may be connected to a thermometer for measuring a temperature of a cell broth in the culture vessel 111, a dissolved oxygen (DO) meter for measuring DO, or a pH meter for measuring pH.

According to the method for producing a product by cells according to the present embodiment, since a product by cells is filtered using the filtration condition acquired in advance, the product by cells can be efficiently obtained.

(Example 1)

A frozen Chinese hamster ovary (CHO) cell line producing a monoclonal antibody (ATCC CRL-12445), which had been obtained by selecting cells adapted to and suspended in a serum-free culture medium, was thawed. Then, the cells were put into a 125 mL Erlenmeyer flask in which 10 mL of a serum-free culture medium having the composition shown in Table 1 had been dispensed in advance, and the cells and a culture medium were mixed in the Erlenmeyer flask. The number of cells was checked with a live and dead cell autoanalyzer (Vi-CELL XR, Beckman Coulter, Inc.), and the cells were diluted with a culture medium so that the cell density was $3.5 \times 10^5$ cells/mL. Thereafter, the cells were shaken and cultured in an incubator at 37°C and in an atmosphere of 5% $CO_2$ for 4 days.

[Table 1]

| Raw material of culture medium composition | Maker | Amount contained in 1 L of culture medium |
|---|---|---|
| EX-CELL® ACF CHO Medium | Sigma-Aldrich | 10.8 g |
| GIBCO™ CD Opti CHO™ AGT™ | Thermo Fisher Scientific | 9.7 g |
| GIBCO™ L-Glutamine 200 mM | Thermo Fisher Scientific | 40 mL |

(continued)

| Raw material of culture medium composition | Maker | Amount contained in 1 L of culture medium |
|---|---|---|
| GIBCO™ MEM Amino Acids [-]L-Glutamine | Thermo Fisher Scientific | 10 mL |
| MEM Vitamin Solution | FUJIFILM Wako Pure Chemical Corporation | 5 mL |
| GIBCO™ HT Supplement | Thermo Fisher Scientific | 5 mL |
| Insulin, Human, recombinant | FUJIFILM Wako Pure Chemical Corporation | 4 mg |
| Kanamycin Sulfate | FUJIFILM Wako Pure Chemical Corporation | 0.05 g |
| Amphotericin B 250 μg/mL | Thermo Fisher Scientific | 1 mL |
| Methotrexate for Biochemistry *used only in cell passage | FUJIFILM Wako Pure Chemical Corporation | 0.2 μmol |

On the 4th day after thawing the cells, 50 mL of a cell broth containing cells at a density of $3.5 \times 10^5$ cells/mL was put into two new 125 mL Erlenmeyer flasks, and the cells were shaken and cultured in an incubator at 37°C and in an atmosphere of 5% $CO_2$ for 3 days. After three days, 120 to 130 mL of a cell broth containing cells at a density of $3.5 \times 10^5$ cells/mL was put into two new 250 mL Erlenmeyer flasks, and the cells were shaken and cultured in an incubator at 37°C and in an atmosphere of 5% $CO_2$ for 3 days. After three days, 500 mL of a cell broth containing cells at a density of $3.5 \times 10^5$ cells/mL was put into three new 1 L Erlenmeyer flasks, and the cells were shaken and cultured in an incubator at 37°C and in an atmosphere of 5% $CO_2$ for 3 days.

6 L of a cell broth containing cells at a density of $5.5 \times 10^5$ cells/mL was put aseptically into a culture vessel having a volume of 12 L, which had been sterilized by autoclaving in advance. The cells were cultured with stirring for 3 days while blowing oxygen so that the DO did not fall below 70% at 37°C in an atmosphere of 5% $CO_2$. After three days, a serum-free culture medium having the composition shown in Table 2 was added to the culture vessel, where this addition was carried out at 0.28 mL/min for 3 days consecutively.

[Table 2]

| Raw material of culture medium to be added | Maker | Amount contained in 1 L of culture medium |
|---|---|---|
| GIBCO™ CHO CD EfficientFeed™ A chemically Defined Feed Supplement | Thermo Fisher Scientific | 450 mL |
| GIBCO™ CHO CD EfficientFeed™ B chemically Defined Feed Supplement | Thermo Fisher Scientific | 450 mL |
| GIBCO™ L-Glutamine 200 mM | Thermo Fisher Scientific | 100 mL |

Next, a cell broth containing cells at a density of $1.5 \times 10^7$ cells/mL was aseptically recovered, and 600 mL of the cell broth was transferred into a spinner flask as a culture vessel which had been sterilized by autoclaving in advance. A mini-module of a porous hollow fiber membrane (manufactured by Asahi Kasei Medical Co., Ltd., BioOptimal MF-SL, blocking pore diameter: 0.4 μm), in which the effective length was adjusted such that the membrane area of the liquid contact portion was 3 $cm^2$, was prepared. The spinner flask and a first opening of the hollow portion, which is the primary side of the porous hollow fiber membrane, were connected to each other by a first flow channel, a second opening of the hollow portion of the porous hollow fiber membrane and the spinner flask were connected to each other by a second flow channel, and the secondary side of the porous hollow fiber membrane and the spinner flask were connected to each other by a third flow channel.

The atmospheric temperature of the spinner flask and the porous hollow fiber membrane was set to 4°C, and the cell broth was stirred in the spinner flask. The cell broth was fed from the spinner flask to the porous hollow fiber membrane through the first flow channel by a magnetic levitation centrifugal pump (Pura Lev i30SU, manufactured by Levitronix), and tangential flow filtration was carried out. A cell broth that had not been filtered through the porous hollow fiber membrane and had passed through the hollow portion of the porous hollow fiber membrane was returned to the spinner flask through the second flow channel. The cell broth filtered through the porous hollow fiber membrane was returned to the spinner flask through the third flow channel. The first and third flow channels had a structure that made it possible to sample the cell broth inside the flow channels.

A single-use pressure gauge (manufactured by PendoTECH, PREPS-N-000 or PREPS-N-012) was installed on the primary side and the secondary side of the porous hollow fiber membrane, and the transmembrane pressure (TMP) was measured over time. The viscosity of the cell broth was measured with an EMS viscometer (EMS-1000S), and the amount of the cell broth fed from the spinner flask to the porous hollow fiber membrane was set such that the shear stress on the inner surface of the porous hollow fiber membrane was 4.58 $N/m^2$. The filtration flow rate in the porous hollow fiber membrane was set to 5 $\mu$L/min by a pump so that the filtration flow rate was constant (1 LMH).

Once or twice a day, the cell broth circulating between the spinner flask and the porous hollow fiber membrane was sampled, and the cell density, the viability of cells, and the concentration of the antibody in the cell broth were measured. As a result, as shown in Figure 3, Figure 5, and Figure 7, each of the cell density, the viability of cells, and the concentration of the antibody in the cell broth was substantially constant. At a time point at which the cell broth was filtered at 300 $L/m^2$ through the BioOptimal MF-SL, the permeation rate of the monoclonal antibody was 78.8%, and the transmembrane pressure was 43.4 kPa in the BioOptimal MF-SL.

(Example 2)

The same method as in Example 1 was carried out, except that a porous hollow fiber membrane Microza UMP manufactured by Asahi Kasei Corporation, having a blocking pore diameter of 0.2 $\mu$m, was used as a mini-module of a porous hollow fiber membrane, in which the effective length was adjusted such that the membrane area of the liquid contact portion was 3 $cm^2$. As a result, as shown in Figure 3, Figure 5, and Figure 7, each of the cell density, the viability of cells, and the concentration of the antibody in the cell broth was substantially constant. At a time point at which the cell broth was filtered at 300 $L/m^2$ through the MICROZA UMP, the permeation rate of the monoclonal antibody was 80.2%, and the transmembrane pressure was 81.0 kPa in the MICROZA UMP.

(Example 3)

The same method as in Example 1 was carried out, except that a porous hollow fiber membrane Microza UJP manufactured by Asahi Kasei Corporation, having a blocking pore diameter of 0.65 $\mu$m, was used as a mini-module of a porous hollow fiber membrane, in which the effective length was adjusted such that the membrane area of the liquid contact portion was 3 $cm^2$. As a result, as shown in Figure 3, Figure 5, and Figure 7, each of the cell density, the viability of cells, and the concentration of the antibody in the cell broth was substantially constant. At a time point at which the cell broth was filtered at 300 $L/m^2$ through the MICROZA UJP, the permeation rate of the monoclonal antibody was 99.2%, and the transmembrane pressure was 1.3 kPa in the MICROZA UJP.

(Example 4)

The same method as in Example 1 was carried out, except that a porous hollow fiber membrane manufactured by Repligen Corporation, having a blocking pore diameter of 0.2 $\mu$m, was used as a mini-module of a porous hollow fiber membrane, in which the effective length was adjusted such that the membrane area of the liquid contact portion was 3 $cm^2$. As a result, as shown in Figure 3, Figure 5, and Figure 7, each of the cell density, the viability of cells, and the concentration of the antibody in the cell broth was substantially constant. At a time point at which the cell broth was filtered at 300 $L/m^2$ with the hollow fiber manufactured by Repligen Corporation, the permeation rate of the monoclonal antibody was 86.4%, and the transmembrane pressure was 1.0 kPa in the hollow fiber manufactured by Repligen Corporation.

(Example 5)

The same method as in Example 1 was carried out, except that a porous hollow fiber membrane manufactured by Cytiva, having a blocking pore diameter of 0.45 $\mu$m, was used as a mini-module of a porous hollow fiber membrane, in which the effective length was adjusted such that the membrane area of the liquid contact portion was 3 $cm^2$. As a result, as shown in Figure 3, Figure 5, and Figure 7, each of the cell density, the viability of cells, and the concentration of the antibody in the cell broth was substantially constant. At a time point at which the cell broth was filtered at 300 $L/m^2$ with the hollow fiber manufactured by Cytiva, the permeation rate of the monoclonal antibody was 80.6%, and the transmembrane pressure was 2.0 kPa in the hollow fiber manufactured by Cytiva.

(Example 6)

A frozen Chinese hamster ovary (CHO) cell line producing a monoclonal antibody (ATCC CRL-12445), which had been obtained by selecting cells adapted to and suspended in a serum-free culture medium, was thawed. Then, the cells were put into a 125 mL Erlenmeyer flask in which 10 mL of a serum-free culture medium having the composition shown in Table 3 had been dispensed in advance, and the cells and a culture medium were mixed in the Erlenmeyer flask. The number of cells was checked with a live and dead cell autoanalyzer (Vi-CELL XR, Beckman Coulter, Inc.), and the cells were diluted with a culture medium so that the cell density was $3.5 \times 10^5$ cells/mL. Thereafter, the cells were shaken and cultured in an incubator at 37°C and in an atmosphere of 5% $CO_2$ for 4 days.

[Table 3]

| Raw material of culture medium composition | Maker | Amount contained in 1 L of culture medium |
|---|---|---|
| EX-CELL® ACF CHO Medium | Sigma-Aldrich | 10.8 g |
| GIBCO™ CD Opti CHO™ AGT™ | Thermo Fisher Scientific | 9.7 g |
| GIBCO™ L-Glutamine 200 mM | Thermo Fisher Scientific | 40 mL |
| GIBCO™ MEM Amino Acids [-]L-Glutamine | Thermo Fisher Scientific | 10 mL |
| MEM Vitamin Solution | FUJIFILM Wako Pure Chemical Corporation | 5 mL |
| GIBCO™ HT Supplement | Thermo Fisher Scientific | 5 mL |
| Insulin, Human, recombinant | FUJIFILM Wako Pure Chemical Corporation | 4 mg |
| Kanamycin Sulfate | FUJIFILM Wako Pure Chemical Corporation | 0.05 g |
| Amphotericin B 250 μg/mL | Thermo Fisher Scientific | 1 mL |
| Methotrexate for Biochemistry *used only in cell passage | FUJIFILM Wako Pure Chemical Corporation | 0.2 μmol |

On the 4th day after thawing the cells, 50 mL of a cell broth containing cells at a density of $3.5 \times 10^5$ cells/mL was put into two new 125 mL Erlenmeyer flasks, and the cells were shaken and cultured in an incubator at 37°C and in an atmosphere of 5% $CO_2$ for 3 days. After three days, 120 to 130 mL of a cell broth containing cells at a density of $3.5 \times 10^5$ cells/mL was put into two new 250 mL Erlenmeyer flasks, and the cells were shaken and cultured in an incubator at 37°C and in an atmosphere of 5% $CO_2$ for 3 days. After three days, 500 mL of a cell broth containing cells at a density of $3.5 \times 10^5$ cells/mL was put into three new 1 L Erlenmeyer flasks, and the cells were shaken and cultured in an incubator at 37°C and in an atmosphere of 5% $CO_2$ for 3 days.

5 L of a cell broth containing cells at a density of $5.5 \times 10^5$ cells/mL was put aseptically into a culture vessel having a volume of 12 L, which had been sterilized by autoclaving in advance. The cells were cultured with stirring for 3 days while blowing oxygen so that the DO did not fall below 70% at 37°C in an atmosphere of 5% $CO_2$. After three days, continuous culture, which was accompanied by filtration with a porous hollow fiber membrane (manufactured by Asahi Kasei Medical Co., Ltd., BioOptimal MF-SL0190, blocking pore diameter: 0.4 μm) and addition of a culture medium to the culture vessel, was started.

A fresh culture medium of the same amount as the amount of the cell broth extracted from the culture vessel by filtration was aseptically fed from a 50 L bag (manufactured by Thermo Fisher Scientific, Inc., Productainer Bioprocess Container (BPC), 50 L) to the culture vessel, and the amount of the cell broth in the culture vessel was controlled to be constant. The culture medium replacement rate of the cell broth was set to 1 vessel volumes per day ($vvd^{-1}$), and the culture medium replacement rate of the cell broth was increased by 0.5 $vvd^{-1}$ at a time point at which the glucose concentration was 1 g/L or less or the glutamine concentration was 1 mmol/L or less.

After 7 days from the start of the continuous culture, a cell broth containing cells at a density of $1 \times 10^8$ cells/mL was aseptically recovered, and 600 mL of the cell broth was transferred into a spinner flask as a culture vessel which had been sterilized by autoclaving in advance. A mini-module of a porous hollow fiber membrane (manufactured by Asahi Kasei Medical Co., Ltd., BioOptimal MF-SL, blocking pore diameter: 0.4 μm), in which the effective length was adjusted such that the membrane area of the liquid contact portion was 3 $cm^2$, was prepared. The spinner flask and a first opening of the hollow portion, which is the primary side of the porous hollow fiber membrane, were connected to each other by a first flow channel, a second opening of the hollow portion of the porous hollow fiber membrane and the spinner flask were connected to each other by a second flow channel, and the secondary side of the porous hollow fiber membrane and the spinner flask were connected to each other by a third flow channel.

The atmospheric temperature of the spinner flask and the porous hollow fiber membrane was set to 4°C, and the cell broth was stirred in the spinner flask. The cell broth was fed from the spinner flask to the porous hollow fiber membrane through the first flow channel by a magnetic levitation centrifugal pump (Pura Lev i30SU, manufactured by Levitronix) , and tangential flow filtration was carried out. A cell broth that had not been filtered through the porous hollow fiber membrane and had passed through the hollow portion of the porous hollow fiber membrane was returned to the spinner flask through the second flow channel. The cell broth filtered through the porous hollow fiber membrane was returned to the spinner flask through the third flow channel. The first and third flow channels had a structure that made it possible to sample the cell broth inside the flow channels.

A single-use pressure gauge (manufactured by PendoTECH, PREPS-N-000 or PREPS-N-012) was installed on the primary side and the secondary side of the porous hollow fiber membrane, and the transmembrane pressure (TMP) was measured over time. The viscosity of the cell broth was measured with an EMS viscometer (EMS-1000S), and the amount of the cell broth fed from the spinner flask to the porous hollow fiber membrane was set such that the shear stress on the inner surface of the porous hollow fiber membrane was 1.69 $N/m^2$. The filtration flow rate in the porous hollow fiber membrane was set to 5 μL/min by a pump so that the filtration flow rate was constant (1 LMH).

Once or twice a day, the cell broth circulating between the spinner flask and the porous hollow fiber membrane was sampled, and the cell density, the viability of cells, and the concentration of the antibody in the cell broth were measured. As a result, as shown in Figure 4, Figure 6, and Figure 8, each of the cell density, the viability of cells, and the concentration of the antibody in the cell broth was substantially constant.

(Example 7)

The same method as in Example 6 was carried out, except that a porous hollow fiber membrane Microza UMP manufactured by Asahi Kasei Corporation, having a blocking pore diameter of 0.2 μm, was used as a mini-module of a porous hollow fiber membrane, in which the effective length was adjusted such that the membrane area of the liquid contact portion was 3 $cm^2$. As a result, as shown in Figure 4, Figure 6, and Figure 8, each of the cell density, the viability of cells, and the concentration of the antibody in the cell broth was substantially constant.

(Example 8)

The same method as in Example 6 was carried out, except that a porous hollow fiber membrane Microza UJP manufactured by Asahi Kasei Corporation, having a blocking pore diameter of 0.65 μm, was used as a mini-module of a porous hollow fiber membrane, in which the effective length was adjusted such that the membrane area of the liquid contact portion was 3 $cm^2$. As a result, as shown in Figure 4, Figure 6, and Figure 8, each of the cell density, the viability of cells, and the concentration of the antibody in the cell broth was substantially constant.

(Example 9)

The same method as in Example 6 was carried out, except that a porous hollow fiber membrane manufactured by Repligen Corporation, having a blocking pore diameter of 0.2 μm, was used as a mini-module of a porous hollow fiber membrane, in which the effective length was adjusted such that the membrane area of the liquid contact portion was 3 $cm^2$. As a result, as shown in Figure 4, Figure 6, and Figure 8, each of the cell density, the viability of cells, and the concentration of the antibody in the cell broth was substantially constant.

(Example 10)

The same method as in Example 6 was carried out, except that a porous hollow fiber membrane manufactured by Cytiva, having a blocking pore diameter of 0.45 μm, was used as a mini-module of a porous hollow fiber membrane, in which the effective length was adjusted such that the membrane area of the liquid contact portion was 3 $cm^2$. As a result, as shown in Figure 4, Figure 6, and Figure 8, each of the cell density, the viability of cells, and the concentration of the antibody in the cell broth was substantially constant.

(Comparative Example 1)

In the same manner as in Examples, 120 to 130 mL of a cell broth containing cells at a density of $3.5 \times 10^5$

cells/mL was put into two new 250 mL Erlenmeyer flasks, and the cells were shaken and cultured in an incubator at 37°C and in an atmosphere of 5% $CO_2$ for 3 days, and then 1.28 L of a cell broth containing cells at a density of $5.5 \times 10^5$ cells/mL was put aseptically into a culture vessel having a volume of 3 L, which had been sterilized by autoclaving in advance. The cells were cultured with stirring for 3 days while blowing oxygen so that the DO did not fall below 70% at 37°C in an atmosphere of 5% $CO_2$. After three days, continuous culture, which was accompanied by filtration with a module of a porous hollow fiber membrane (manufactured by Asahi Kasei Medical Co., Ltd., BioOptimal MF-SL, blocking pore diameter: 0.4 μm), in which the number of sheets and the effective length were adjusted such that the membrane area of the liquid contact portion was 200 $cm^2$, and addition of a culture medium to the culture vessel, was started.

The culture vessel and a first opening of the hollow portion, which is the primary side of the porous hollow fiber membrane, were connected to each other by a first flow channel, and a second opening of the hollow portion of the porous hollow fiber membrane and the culture vessel were connected to each other by a second flow channel. The cell broth filtered through the porous hollow fiber membrane was not allowed to return to the culture vessel. In addition, the culture vessel was equipped with an outflow port for sampling an internal cell broth and a supply port for supplying a fresh culture medium.

A fresh culture medium of the same amount as the amount of the cell broth extracted from the culture vessel by filtration was aseptically fed from a 20 L bag (manufactured by Thermo Fisher Scientific, Inc., Productainer Bioprocess Container (BPC), 20 L) to the culture vessel, and the amount of the cell broth in the culture vessel was controlled to be constant. The culture medium replacement rate of the cell broth was set to 1 vessel volumes per day ($vvd^{-1}$), and the culture medium replacement rate of the cell broth was increased by 0.375 $vvd^{-1}$ at a time point at which the glucose concentration was 1 g/L or less or the glutamine concentration was 1 mmol/L or less.

The temperature of the cell broth in the culture vessel was controlled to 37°C, and the cell broth was stirred in the culture vessel. The cell broth was fed from the culture vessel to the porous hollow fiber membrane through the first flow channel by a magnetic levitation centrifugal pump (Pura Lev 200MU, manufactured by Levitronix), and tangential flow filtration was carried out. A cell broth that had not been filtered through the porous hollow fiber membrane and had passed through the hollow portion of the porous hollow fiber membrane was returned to the culture vessel through the second flow channel. In a case where the DO of the cell broth was less than 70%, oxygen was introduced into the cell broth in the culture vessel using a sparger. In addition, air containing carbon dioxide at a concentration of 5% was at all times introduced into the culture vessel.

A single-use pressure gauge (manufactured by PendoTECH, PREPS-N-038) was installed on the primary side and the secondary side of the porous hollow fiber membrane, and the transmembrane pressure (TMP) was measured over time. The amount of the cell broth fed from the culture vessel to the porous hollow fiber membrane was set such that the shear stress on the inner surface of the porous hollow fiber membrane was 2.10 $N/m^2$. The filtration flow rate in the porous hollow fiber membrane was increased stepwise to 1.0 LMH, 1.5 LMH, 2.0 LMH, 2.5 LMH, and 3.0 LMH by a pump, and the introduction amount of the fresh culture medium was allowed to be increased for adjusting the glucose concentration and the glutamine concentration in accordance with the increase in the number of cells. In addition, bleeding was carried out so that the cell density in the cell broth in the culture vessel was $7.5 \times 10^7$ cells/mL. Further, a liquid level sensor was disposed in the culture vessel, and a fresh culture medium was introduced into the culture vessel so that the liquid level was constant.

Once or twice a day, the cell broth in the culture vessel was sampled, and the cell density, the viability of cells, and the concentration of the antibody in the cell broth were measured. As a result, as shown in Figure 9, the cell density in the cell broth continued to increase until the bleeding started. As shown in Figure 10, the viability of cells was substantially constant. As shown in Figure 11, the concentration of the antibody tended to increase.

(Comparative Example 2)

The same method as in Comparative Example 1 was carried out, except that a porous hollow fiber membrane Microza UMP manufactured by Asahi Kasei Corporation, having a blocking pore diameter of 0.2 μm, was used as a module of a porous hollow fiber membrane, in which the number of sheets and the effective length were adjusted such that the membrane area of the liquid contact portion was 200 $cm^2$.

Once or twice a day, the cell broth in the culture vessel was sampled, and the cell density, the viability of cells, and the concentration of the antibody in the cell broth were measured. As a result, as shown in Figure 9, the cell density in the cell broth continued to increase until the bleeding started. As shown in Figure 10, the viability of cells was substantially constant. As shown in Figure 11, the concentration of the antibody tended to increase.

(Reference Examples 1 to 3)

A cell broth containing cells at a density of $1.5 \times 10^7$ cells/mL, which had been prepared in the same manner as in Example 1, was aseptically recovered, and 600 mL of the cell broth was transferred into a spinner flask as a culture vessel which had been sterilized by autoclaving in advance.

The atmospheric temperature of the spinner flask was set to 4°C, and the cell broth was stirred in the spinner flask. The hollow fiber membrane was not connected to the spinner flask, and the cells were cultured only in the spinner flask. Once or twice a day, the cell broth in the spinner flask was sampled, and the cell density, the viability of cells, and the concentration of the antibody in the cell broth were measured. An experiment under the same conditions as described above except that the atmospheric temperature was set to room temperature (RT) and an experiment under the same conditions as described above except that the atmospheric temperature was set to 37°C were also carried out. As a result, as shown in Figure 12, the cell density tended to increase at room temperature. As shown in Figure 13, the viability of cells tended to decrease at room temperature and 37°C. As shown in Figure 14, the concentration of the antibody tended to increase at room temperature and 37°C.

(Reference Examples 4 to 6)

A cell broth containing cells at a density of $1.5 \times 10^7$ cells/mL, which had been prepared in the same manner as in Example 1, was aseptically recovered, and 600 mL of the cell broth was transferred into a spinner flask as a culture vessel which had been sterilized by autoclaving in advance.
The atmospheric temperature of the spinner flask was set to 8°C, and the cell broth was stirred in the spinner flask. The hollow fiber membrane was not connected to the spinner flask, and the cells were cultured only in the spinner flask. Once or twice a day, the cell broth in the spinner flask was sampled, and the cell density, the viability of cells, and the concentration of the antibody in the cell broth were measured. An experiment under the same conditions as described above except that the atmospheric temperature was set to 12°C and an experiment under the same conditions as described above except that the atmospheric temperature was set to 37°C were also carried out. As a result, as shown in Figure 15, the cell density tended to decrease at 37°C. As shown in Figure 16, the viability of cells tended to decrease at 37°C.

(Reference Examples 7 and 8)

A cell broth containing cells at a density of $1.5 \times 10^7$ cells/mL, which had been prepared in the same manner as in Example 1, was aseptically recovered, and 600 mL of the cell broth was transferred into a spinner flask as a culture vessel which had been sterilized by autoclaving in advance.
The atmospheric temperature of the spinner flask was set to 4°C, and the cell broth was stirred in the spinner flask. The hollow fiber membrane was not connected to the spinner flask, and the cells were cultured only in the spinner flask. Once or twice a day, the cell broth in the spinner flask was sampled, and the cell density, the viability of cells, and the concentration of the antibody in the cell broth were measured. An experiment was also carried out under the same conditions as described above except that the cell broth was circulated using a pump. The cell broth was fed from the spinner flask to the pump through the first flow channel by a magnetic levitation centrifugal pump (Pura Lev i30SU, manufactured by Levitronix) , and the cell broth that had passed through the pump was returned to the spinner flask through the second flow channel. The cell broth was fed by a pump at 50 mL/min. The first flow channel had a structure that made it possible to sample the cell broth inside the flow channel. Once or twice a day, the cell broth in the spinner flask was sampled from the first flow channel, and the cell density, the viability of cells, and the concentration of the antibody in the cell broth were measured. As a result, as shown in Figure 17 to Figure 19, the temporal change of the cell broth was suppressed in the same manner in a case where the cell broth was circulated by the pump and in a case where the cell broth was not circulated by the pump.

(Analysis method)

The analysis methods used in Examples, Comparative Examples, and Reference Examples will be described below. The density and viability of cells contained in the cell broth were measured by a live and dead cell autoanalyzer (Vi-CELL XR, manufactured by Beckman Coulter, Inc.). The sample was diluted using PBS (-) (FUJIFILM Wako Pure Chemical Corporation), and 600 μL thereof was used for analysis. A method, in which "CHO" in Vi-CELL XR was used and the set values of a Minimum diameter (μm), a Cell brightness (%), and a Viable cell spot brightness (%) were changed as appropriate according to the actual situation, was used as an image analysis method.
The HPLC measurement of the antibody concentration was carried out by the following method.

(1) Detector: Ultraviolet absorption spectrophotometer (measurement wavelength: 280 nm)
(2) Column: POROS G 20 μm Column, $4.6 \times 50$ mm, 0.8 mL (Thermo Fisher Scientific, Inc.)
(3) Column temperature: room temperature
(4) Mobile phase

Mobile phase A: 7.098 g of disodium hydrogen phosphate (anhydrous) and 8.766 g of sodium chloride were dissolved in 800 mL of water, 1 mol/L hydrochloric acid was added thereto to adjust the pH to 7.0, and then water was added thereto to adjust the total volume to 1,000 mL.
Mobile phase B: 12 mL of 1 mol/L hydrochloric acid and 8.766 g of sodium chloride were dissolved in water, and the total volume was adjusted to 1,000 mL.

(5) Feeding of mobile phase

The proportion of the mobile phase A and the mobile phase B was changed as shown in Table 4 below, and then feeding was carried out at a flow rate of 2 mL/min.

[Table 4]

| Time after specimen injection (minutes) | Mobile phase A (vol%) | Mobile phase B (vol%) |
|---|---|---|
| 0 to 4 | 100 | 0 |
| 4 to 11 | 0 | 100 |
| 11 to 16 | 100 | 0 |

The cell broth was centrifuged at 300 × g for 2 minutes, and then the supernatant was sampled. A series of nine stepwise diluted solutions of a commercially available human immunoglobulin G (blood donation Venoglobulin IH 5% for intravenous injection, manufactured by the Japan Blood Products Organization, 2.5 g/50 mL) and the supernatant of the cell broth were fed according to the above procedure. A standard curve was created using 9 peak areas of human immunoglobulin G, and then the antibody concentration in each solution was calculated from the standard curve and the peak area of the specimen.
A shear stress SS due to the cell broth on the liquid contact surface of the hollow fiber membrane is given by a product of a viscosity VC (Pa·s) of a cell broth flowing through the hollow portion of the hollow fiber membrane and a shear rate SV (/s), as shown in the following expression.

$$SS = VC \times SV$$

The shear rate can be calculated from the linear velocity and the diameter of the flow channel, and the linear velocity can be acquired from the pump output. Therefore, the shear stress was calculated based on the viscosity of the cell broth and the shear rate calculated from the linear velocity.

Reference Signs List

11, 111: culture vessel
12, 112: hollow fiber membrane
13, 14, 15, 113, 114, 115, 116, 117, 118, 119: flow channel
16: stirring device
23, 25, 123, 125, 126: pump
33, 34, 35, 133, 134, 135: pressure gauge
50: temperature management chamber
201: container
216: culture medium reservoir
217: container
218: container

**Claims**

**1.** An evaluation method of a filtration condition, the evaluation method comprising:

under a condition in which proliferation of cells is suppressed, feeding a cell broth containing cells from a culture vessel containing the cell broth to a porous membrane, returning a cell broth that has passed through the porous membrane without being filtered, to the culture vessel, and circulating the cell broth between the culture vessel and the porous membrane; and
evaluating one or more filtration conditions with the porous membrane.

**2.** The evaluation method of a filtration condition according to Claim 1,
wherein, in the returning of the cell broth that has passed through the porous membrane without being filtered to the culture vessel, the cell broth that has passed through the porous membrane without being filtered and a cell broth that has been filtered through the porous membrane are returned to the culture vessel.

**3.** The evaluation method of a filtration condition according to Claim 1, further comprising:

evaluating filtration performance of the porous membrane under the one or more filtration conditions,
wherein the one or more filtration conditions with the porous membrane are evaluated based on the filtration performance of the porous membrane.

**4.** The evaluation method of a filtration condition according to Claim 1,
wherein the condition in which the proliferation of cells is suppressed is a condition in which cell cycle progression is suppressed.

**5.** The evaluation method of a filtration condition according to Claim 1,
wherein the condition in which the proliferation of cells is suppressed is at least one of an atmospheric temperature or a cell broth temperature, which is 15°C or lower.

**6.** The evaluation method of a filtration condition according to Claim 5,
wherein the at least one of the atmospheric temperature or the cell broth temperature, which is 15°C or lower, is at least one of an atmospheric temperature or a cell broth temperature, which is 10°C or lower or 5°C or lower.

**7.** The evaluation method of a filtration condition according to Claim 1,
wherein the condition in which the proliferation of cells is suppressed is a condition in which an activity of an enzyme of the cells is suppressed.

**8.** The evaluation method of a filtration condition according to Claim 7,
wherein the enzyme is a cyclin-dependent kinase.

**9.** The evaluation method of a filtration condition according to Claim 1,
wherein the condition in which the proliferation of cells is suppressed is a presence of a cell cycle inhibitor in the cell broth.

**10.** The evaluation method of a filtration condition according to Claim 1,
wherein the one or more filtration conditions with the porous membrane are evaluated based on a permeation rate of a product by the cells in the porous membrane.

**11.** The evaluation method of a filtration condition according to Claim 1,
wherein the one or more filtration conditions with the porous membrane are evaluated based on a permeation flux of the porous membrane.

**12.** The evaluation method of a filtration condition according to Claim 1,
wherein the one or more filtration conditions with the porous membrane are evaluated based on a transmembrane pressure of the porous membrane.

**13.** The evaluation method of a filtration condition according to Claim 1,
wherein the one or more filtration conditions with the porous membrane are evaluated based on a turbidity of a filtrate.

**14.** The evaluation method of a filtration condition according to Claim 1,
wherein the one or more filtration conditions are one condition or a plurality of conditions of a structure, a material, or physical properties of the porous membrane.

**15.** The evaluation method of a filtration condition according to Claim 1,

wherein the one or more filtration conditions are one condition or a plurality of conditions of the cell broth.

**16.** The evaluation method of a filtration condition according to Claim 1,
wherein the one or more filtration conditions are one condition or a plurality of conditions of a cell density in the cell broth.

**17.** The evaluation method of a filtration condition according to Claim 1,
wherein the one or more filtration conditions are one condition or a plurality of conditions of a flow rate of the cell broth which is fed to the porous membrane.

**18.** The evaluation method of a filtration condition according to Claim 1,
wherein the one or more filtration conditions are one condition or a plurality of conditions of a flow rate of the cell broth that has been filtered through the porous membrane.

**19.** The evaluation method of a filtration condition according to Claim 1,
wherein the one or more filtration conditions are one condition or a plurality of conditions of a shear stress on a liquid contact surface of the porous membrane due to a flow of the cell broth which is fed to the porous membrane.

**20.** The evaluation method of a filtration condition according to Claim 1,
wherein a path including the culture vessel and the porous membrane through which the cell broth circulates is disposed in a temperature management chamber.

**21.** The evaluation method of a filtration condition according to Claim 1,
wherein, in circulating the cell broth between the culture vessel and the porous membrane, an active operation for maintaining a composition of the cell broth is not performed.

**22.** The evaluation method of a filtration condition according to Claim 1,
wherein, in circulating the cell broth between the culture vessel and the porous membrane, a culture medium is not added from an outside to a path including the culture vessel and the porous membrane through which the cell broth circulates.

**23.** The evaluation method of a filtration condition according to Claim 1,
wherein, in circulating the cell broth between the culture vessel and the porous membrane, at least one of dissolved oxygen or pH of the cell broth is not controlled.

**24.** The evaluation method of a filtration condition according to Claim 1,
wherein, in circulating the cell broth between the culture vessel and the porous membrane, the cells are not bled from a path including the culture vessel and the porous membrane through which the cell broth circulates.

**25.** The evaluation method of a filtration condition according to Claim 1, further comprising:
in circulating the cell broth between the culture vessel and the porous membrane, collecting the cell broth for sampling from a path including the culture vessel and the porous membrane through which the cell broth circulates.

**26.** The evaluation method of a filtration condition according to Claim 25, further comprising:
measuring a cell density in the sampled cell broth.

**27.** The evaluation method of a filtration condition according to Claim 25, further comprising:
measuring a viability of the cells in the sampled cell broth.

**28.** The evaluation method of a filtration condition according to Claim 25, further comprising:
measuring a concentration of a product by the cells in the sampled cell broth.

**29.** The evaluation method of a filtration condition according to Claim 25, further comprising:
measuring a turbidity of the cell broth in the sampled cell broth.

**30.** The evaluation method of a filtration condition according to Claim 1,
wherein the porous membrane is a hollow fiber membrane.

**31.** The evaluation method of a filtration condition according to Claim 1,
wherein the porous membrane is a microfiltration membrane.

**32.** A method for producing a product by cells, the method comprising:

under a condition in which cells are proliferated, feeding a cell broth containing cells from a culture vessel containing the cell broth to a porous membrane using a filtration condition, returning a cell broth that has passed through the porous membrane without being filtered, to the culture vessel, recovering a cell broth containing a product by the cells, the cell broth having been filtered through the porous membrane, and circulating at least a part of the cell broth between the culture vessel and the porous membrane,
wherein the filtration condition is a filtration condition obtained by, under a condition in which the proliferation of cells is suppressed, feeding the cell broth containing the cells from the culture vessel containing the cell broth to the porous membrane, returning a cell broth that has passed through the porous membrane without being filtered, to the culture vessel, circulating the cell broth between the culture vessel and the porous membrane, and evaluating one or more filtration conditions with the porous membrane.

**33.** The method for producing a product by cells according to Claim 32,
wherein, in a case of evaluating the one or more filtration conditions with the porous membrane, in the returning of the cell broth that has passed through the porous membrane without being filtered to the culture vessel, the cell broth that has passed through the porous membrane without being filtered and a cell broth that has been filtered through the porous membrane are returned to the culture vessel.

**34.** The method for producing a product by cells according to Claim 32,
wherein the condition in which the cells are proliferated is a condition in which a cell cycle progresses.

**35.** The method for producing a product by cells according to Claim 32,
wherein the condition in which the cells are proliferated is at least one of an atmospheric temperature or a cell broth temperature, which is 15°C or more.

**36.** The method for producing a product by cells according to Claim 35,
wherein the at least one of the atmospheric temperature or the cell broth temperature, which is higher than 15°C is at least one of an atmospheric temperature or a cell broth temperature, which is higher than 20°C, 25°C, 30°C, or 35°C.

**37.** The method for producing a product by cells according to Claim 32,
wherein the condition in which the cells are proliferated is a condition in which an enzyme of the cells is activated.

**38.** The method for producing a product by cells according to Claim 37,
wherein the enzyme is a cyclin-dependent kinase.

**39.** The method for producing a product by cells according to Claim 32,
wherein the condition in which the cells are proliferated is an absence of a cell cycle inhibitor in the cell broth.

**40.** The method for producing a product by cells according to Claim 32,
wherein the condition in which the proliferation of cells is suppressed is a condition in which cell cycle progression is suppressed.

**41.** The method for producing a product by cells according to Claim 32,
wherein the condition in which the proliferation of cells is suppressed is at least one of an atmospheric temperature or a cell broth temperature, which is 15°C or lower.

**42.** The method for producing a product by cells according to Claim 41,
wherein the at least one of the atmospheric temperature or the cell broth temperature, which is 15°C or lower, is at least one of an atmospheric temperature or a cell broth temperature, which is 10°C or lower or 5°C or lower.

**43.** The method for producing a product by cells according to Claim 32,
wherein the condition in which the proliferation of cells is suppressed is a condition in which an activity of an enzyme of the cells is suppressed.

**44.** The method for producing a product by cells according to Claim 43, wherein the enzyme is a cyclin-dependent kinase.

**45.** The method for producing a product by cells according to Claim 32, wherein the condition in which the proliferation of cells is suppressed is a presence of a cell cycle inhibitor in the cell broth.

**46.** The method for producing a product by cells according to Claim 32, wherein the one or more filtration conditions with the porous membrane have been evaluated based on a permeation rate of the product by the cells in the porous membrane.

**47.** The method for producing a product by cells according to Claim 32, wherein the one or more filtration conditions with the porous membrane have been evaluated based on a permeation flux of the porous membrane.

**48.** The method for producing a product by cells according to Claim 32, wherein the one or more filtration conditions with the porous membrane have been evaluated based on a transmembrane pressure of the porous membrane.

**49.** The method for producing a product by cells according to Claim 32, wherein the one or more filtration conditions with the porous membrane have been evaluated based on a turbidity of a filtrate.

**50.** The method for producing a product by cells according to Claim 32, wherein the one or more filtration conditions are one condition or a plurality of conditions of a structure, a material, or physical properties of the porous membrane.

**51.** The method for producing a product by cells according to Claim 32, wherein the one or more filtration conditions are one condition or a plurality of conditions of the cell broth.

**52.** The method for producing a product by cells according to Claim 32, wherein the one or more filtration conditions are one condition or a plurality of conditions of a cell density in the cell broth.

**53.** The method for producing a product by cells according to Claim 32, wherein the one or more filtration conditions are one condition or a plurality of conditions of a flow rate of the cell broth which is fed to the porous membrane.

**54.** The method for producing a product by cells according to Claim 32, wherein the one or more filtration conditions are one condition or a plurality of conditions of a flow rate of the cell broth that has been filtered through the porous membrane.

**55.** The method for producing a product by cells according to Claim 32, wherein the one or more filtration conditions are one condition or a plurality of conditions of a shear stress on a liquid contact surface of the porous membrane due to a flow of the cell broth which is fed to the porous membrane.

**56.** The method for producing a product by cells according to Claim 32, wherein, in a case of evaluating the one or more filtration conditions with the porous membrane, a path including the culture vessel and the porous membrane through which the cell broth circulates is disposed in a temperature management chamber.

**57.** The method for producing a product by cells according to Claim 32, wherein, in a case of evaluating the one or more filtration conditions with the porous membrane, in circulating the cell broth between the culture vessel and the porous membrane, an active operation for maintaining a composition of the cell broth is not performed.

**58.** The method for producing a product by cells according to Claim 32, wherein, in a case of evaluating the one or more filtration conditions with the porous membrane, in circulating the cell broth between the culture vessel and the porous membrane, a culture medium is not added from an outside to a path through which the cell broth circulates.

**59.** The method for producing a product by cells according to Claim 32, wherein, in a case of evaluating the one or more filtration conditions with the porous membrane, in circulating the cell broth between the culture vessel and the porous membrane, at least one of dissolved oxygen or pH of the cell broth is not controlled.

**60.** The method for producing a product by cells according to Claim 32, wherein, in a case of evaluating the one or more filtration conditions with the porous membrane, in circulating the cell broth between the culture vessel and the porous membrane, the cells are not bled from a path including the culture vessel and the porous membrane through which the cell broth circulates.

**61.** The method for producing a product by cells according to Claim 32, wherein, in a case of evaluating the one or more filtration conditions with the porous membrane, in circulating the cell broth between the culture vessel and the porous membrane, the method includes collecting the cell broth for sampling from a path including the culture vessel and the porous membrane through which the cell broth circulates.

**62.** The method for producing a product by cells according to Claim 61, further comprising: measuring a cell density in the sampled cell broth.

**63.** The method for producing a product by cells according to Claim 61, further comprising: measuring a viability of the cells in the sampled cell broth.

**64.** The method for producing a product by cells according to Claim 61, further comprising: measuring a concentration of the product by the cells in the sampled cell broth.

**65.** The method for producing a product by cells according to Claim 61, further comprising: measuring a turbidity of the cell broth in the sampled cell broth.

**66.** The method for producing a product by cells according to Claim 32, wherein the porous membrane is a hollow fiber membrane.

**67.** The method for producing a product by cells according to Claim 32, wherein the porous membrane is a microfiltration membrane.

**68.** The method for producing a product by cells according to Claim 32, wherein the cells are subjected to perfusion culture.

Fig. 1

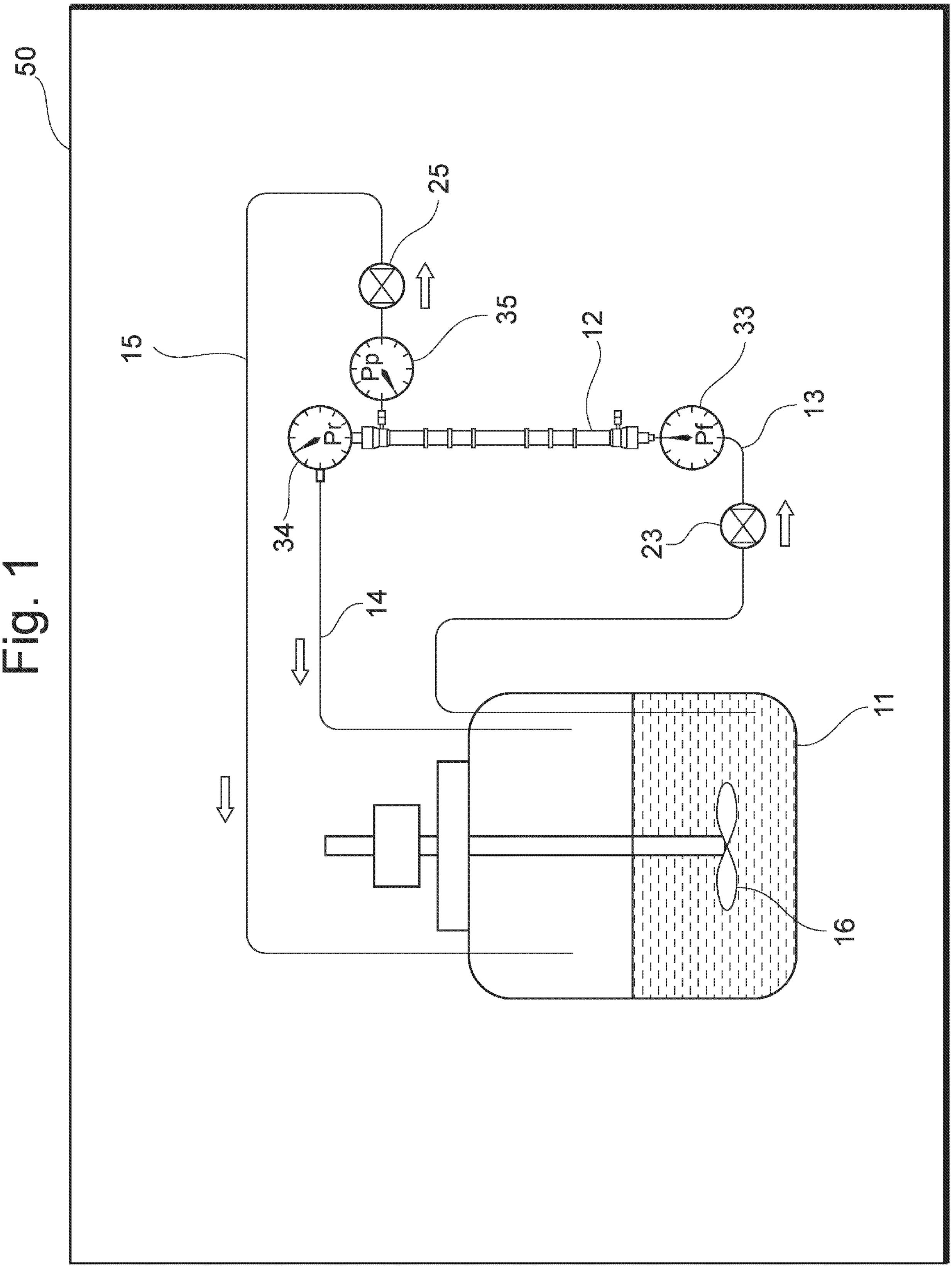

Fig. 2

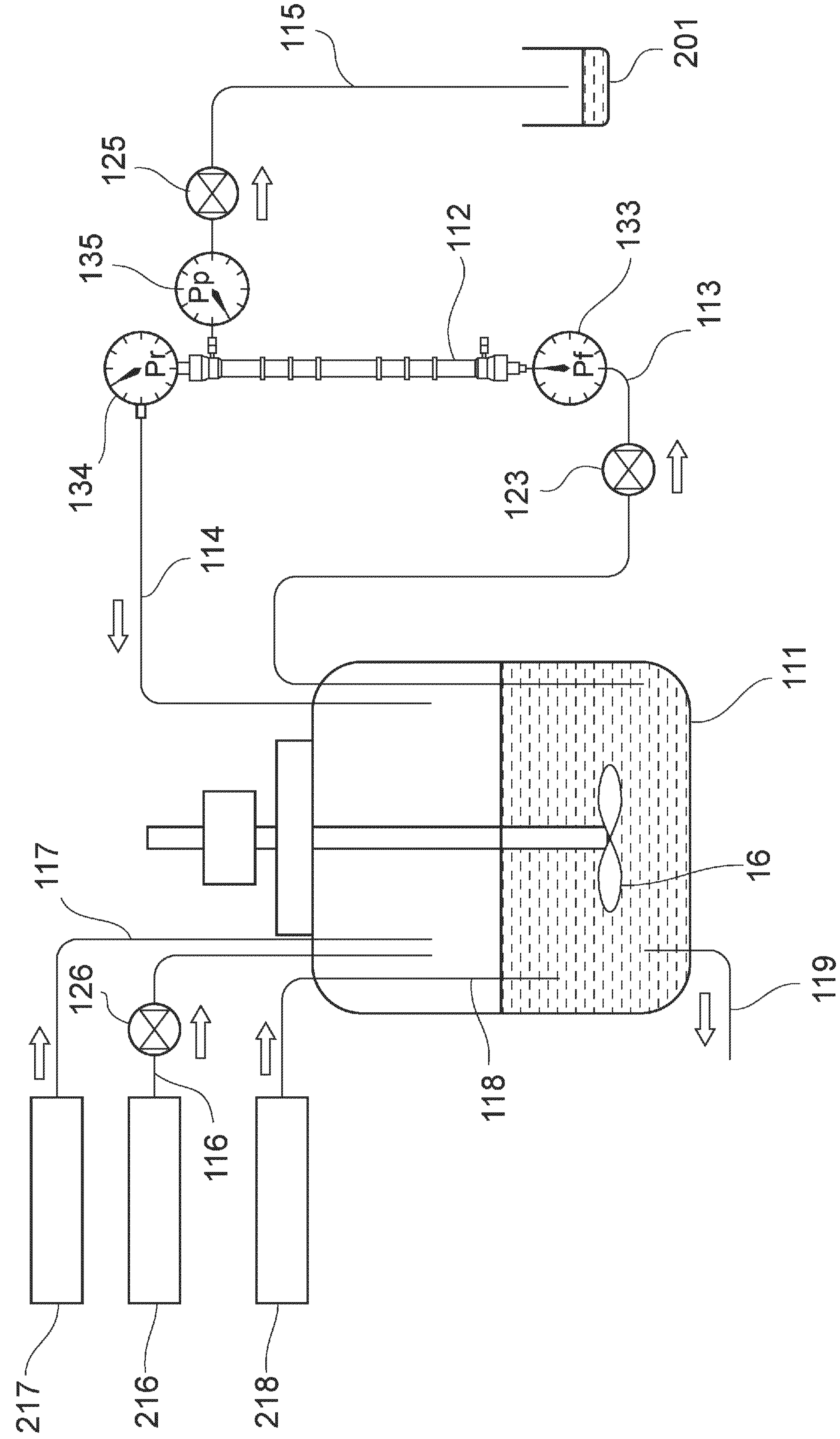

115
201
125
135
Pp
112
133
113
Pr
Pf
134
123
114
111
117
16
126
119
118
116
217
216
218

Fig. 3

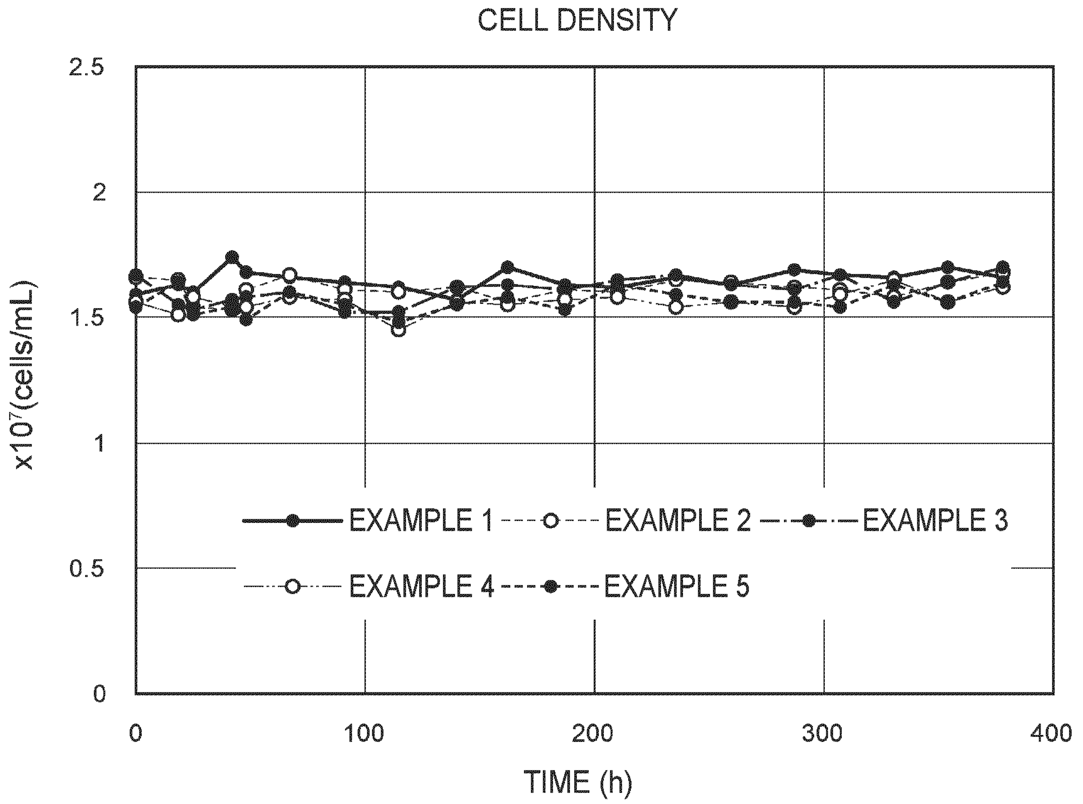
CELL DENSITY
2.5
2
1.5
1
0.5
0
x10⁷(cells/mL)
EXAMPLE 1
EXAMPLE 2
EXAMPLE 3
EXAMPLE 4
EXAMPLE 5
0
100
200
300
400
TIME (h)

Fig. 4

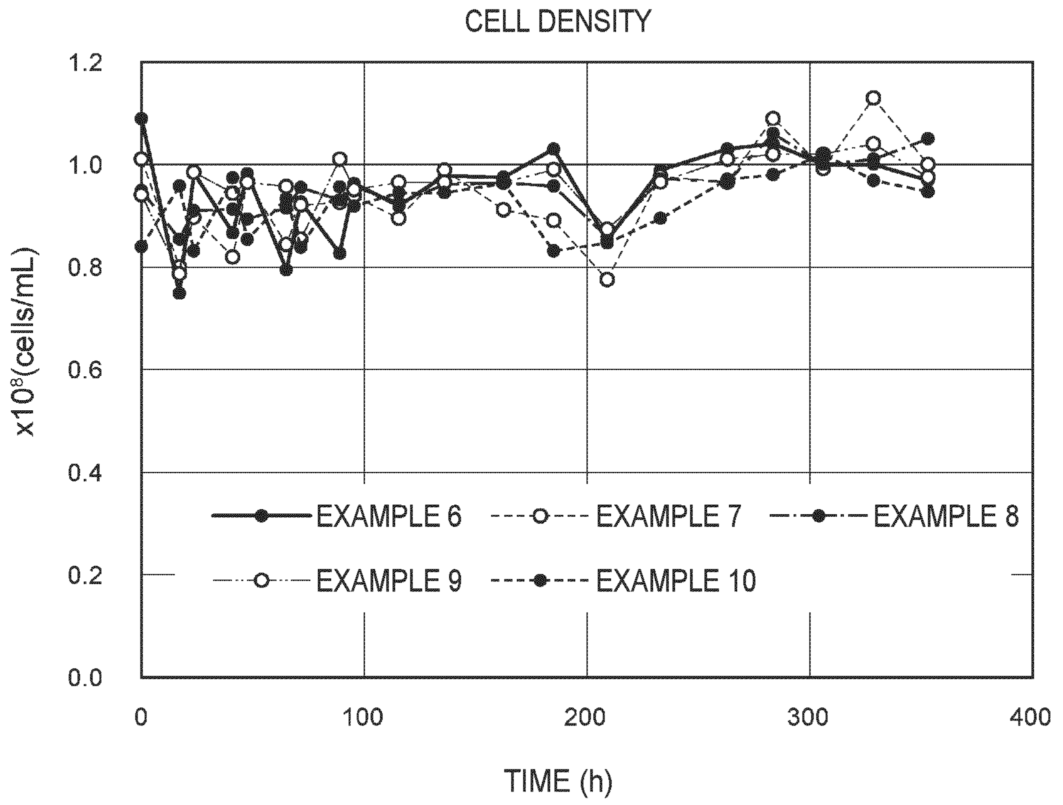
CELL DENSITY
1.2
1.0
0.8
0.6
0.4
0.2
0.0
x10⁸(cells/mL)
EXAMPLE 6
EXAMPLE 7
EXAMPLE 8
EXAMPLE 9
EXAMPLE 10
0
100
200
300
400
TIME (h)

Fig. 5

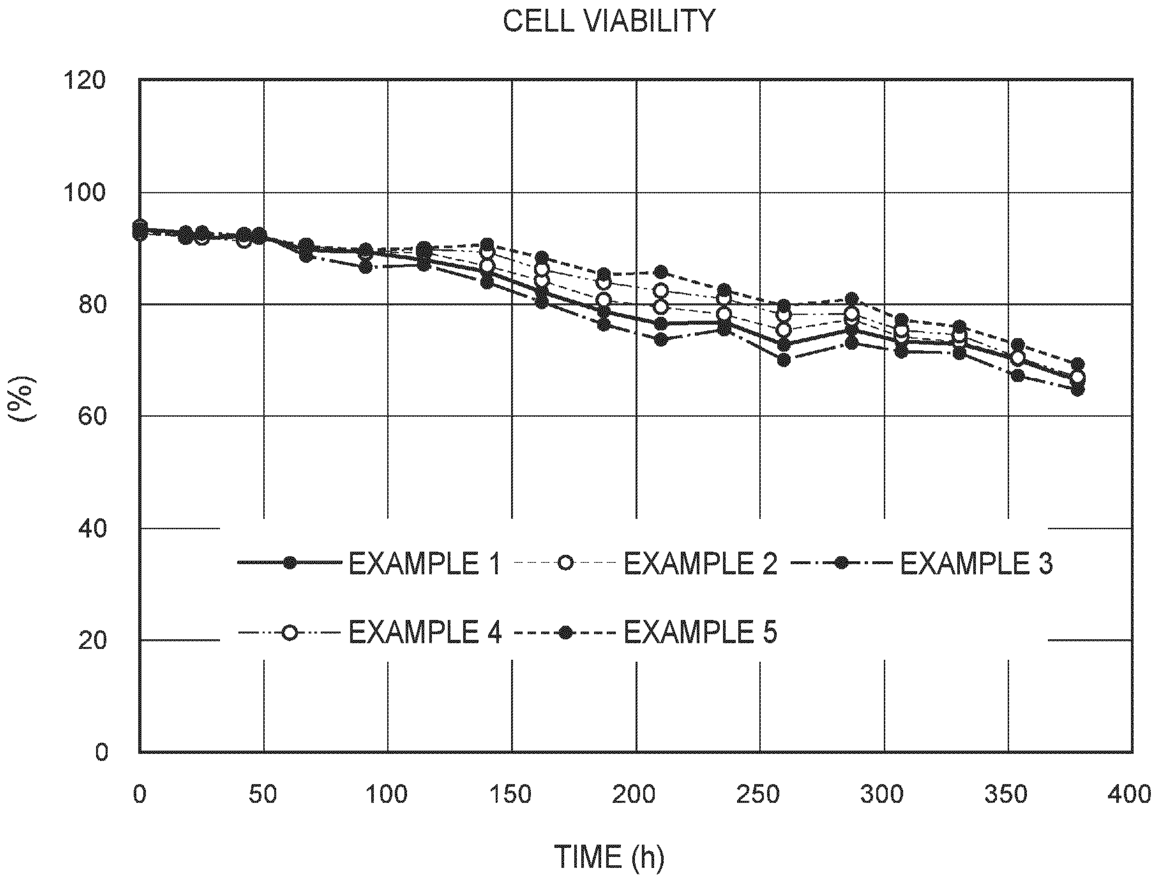
CELL VIABILITY
120
100
80
60
40
20
0
(%)
0
50
100
150
200
250
300
350
400
TIME (h)
EXAMPLE 1
EXAMPLE 2
EXAMPLE 3
EXAMPLE 4
EXAMPLE 5

Fig. 6

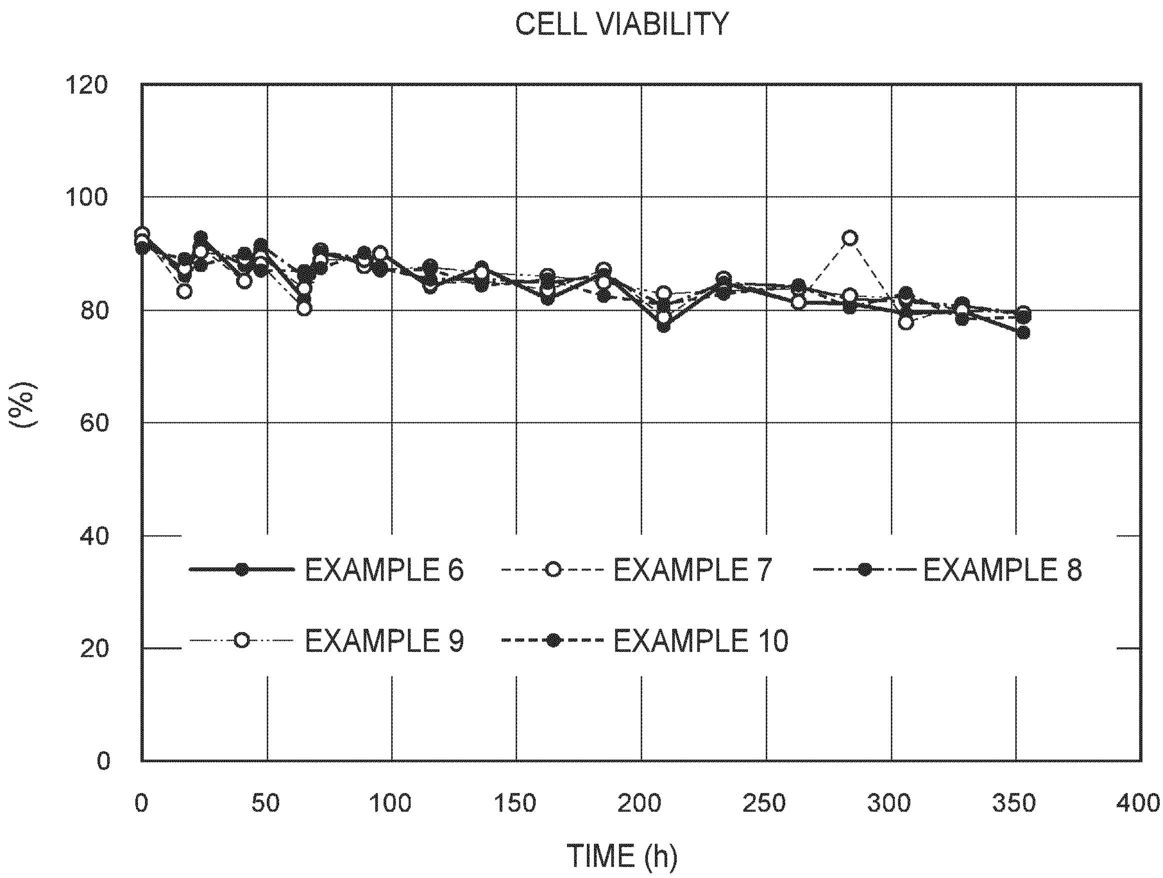
CELL VIABILITY
120
100
80
60
40
20
0
(%)
0
50
100
150
200
250
300
350
400
TIME (h)
EXAMPLE 6
EXAMPLE 7
EXAMPLE 8
EXAMPLE 9
EXAMPLE 10

Fig. 7

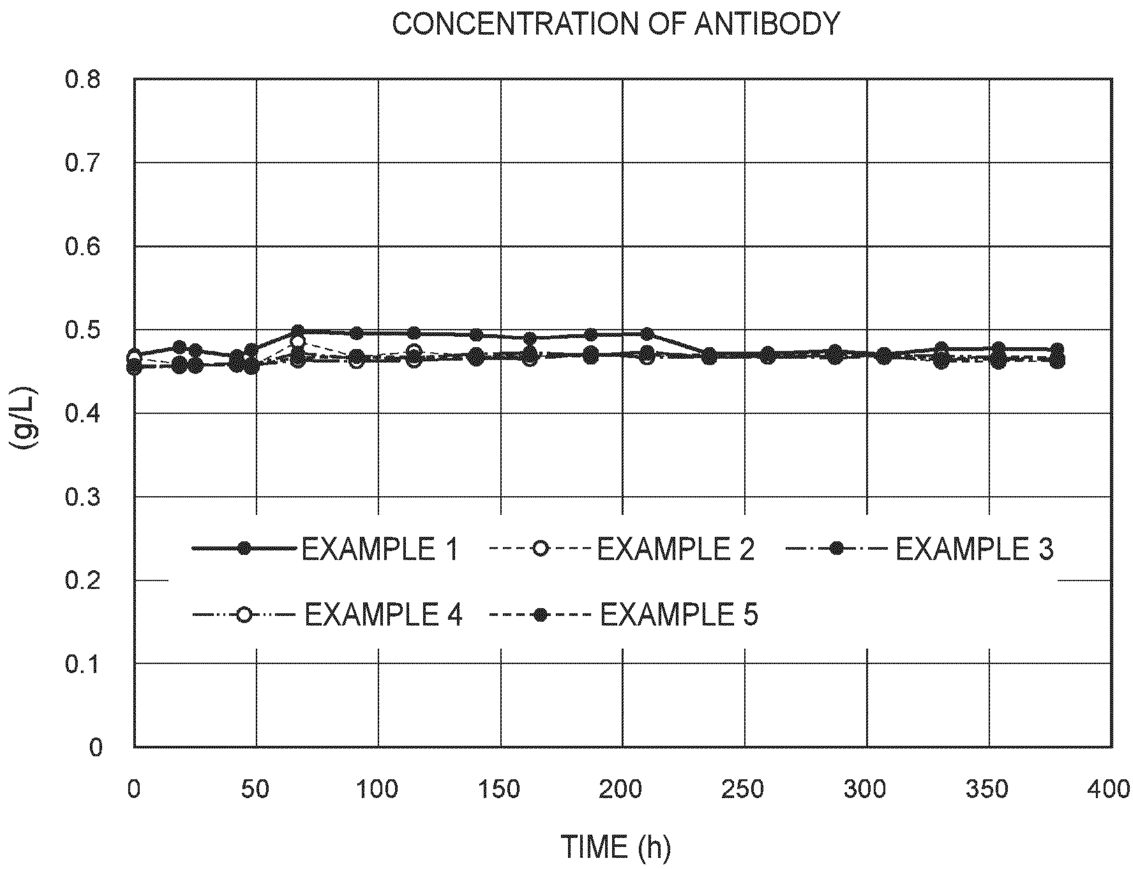
CONCENTRATION OF ANTIBODY
0.8
0.7
0.6
0.5
0.4
0.3
0.2
0.1
0
(g/L)
0
50
100
150
200
250
300
350
400
TIME (h)
EXAMPLE 1
EXAMPLE 2
EXAMPLE 3
EXAMPLE 4
EXAMPLE 5

Fig. 8

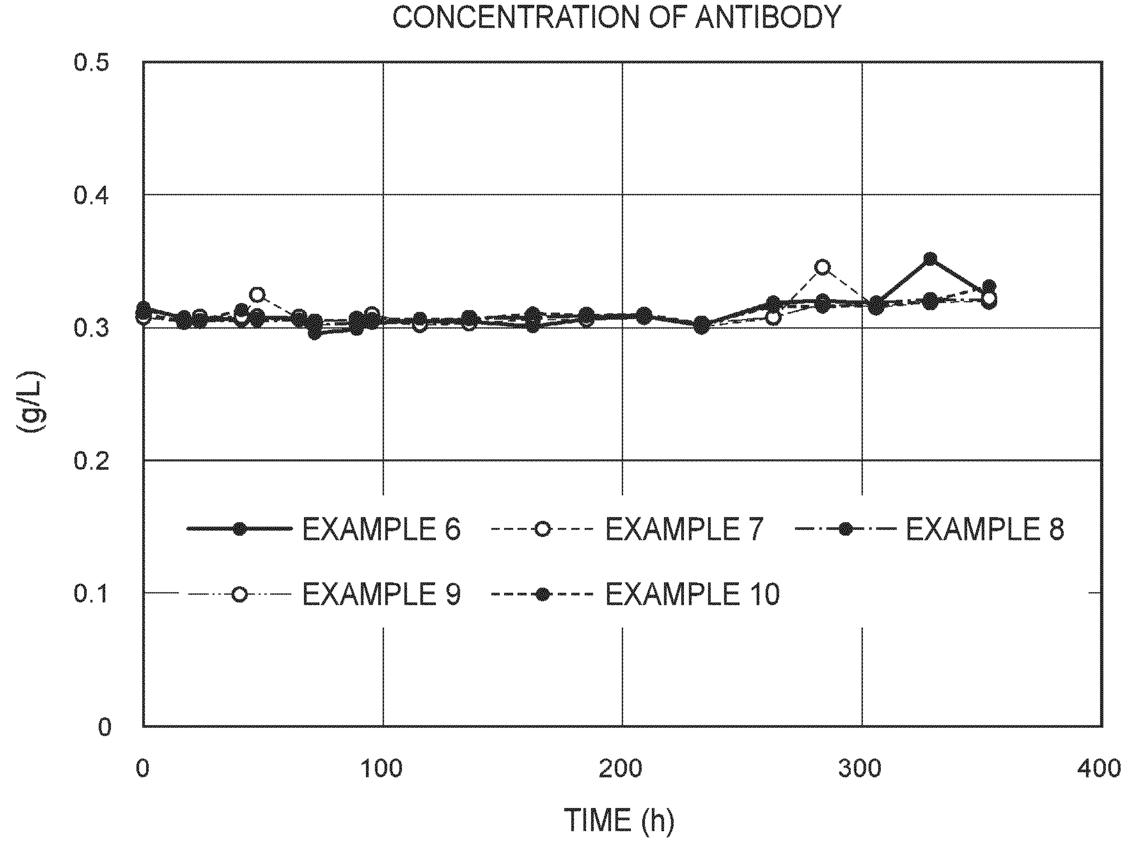
CONCENTRATION OF ANTIBODY
0.5
0.4
0.3
0.2
0.1
0
(g/L)
EXAMPLE 6
EXAMPLE 7
EXAMPLE 8
EXAMPLE 9
EXAMPLE 10
0
100
200
300
400
TIME (h)

Fig. 9

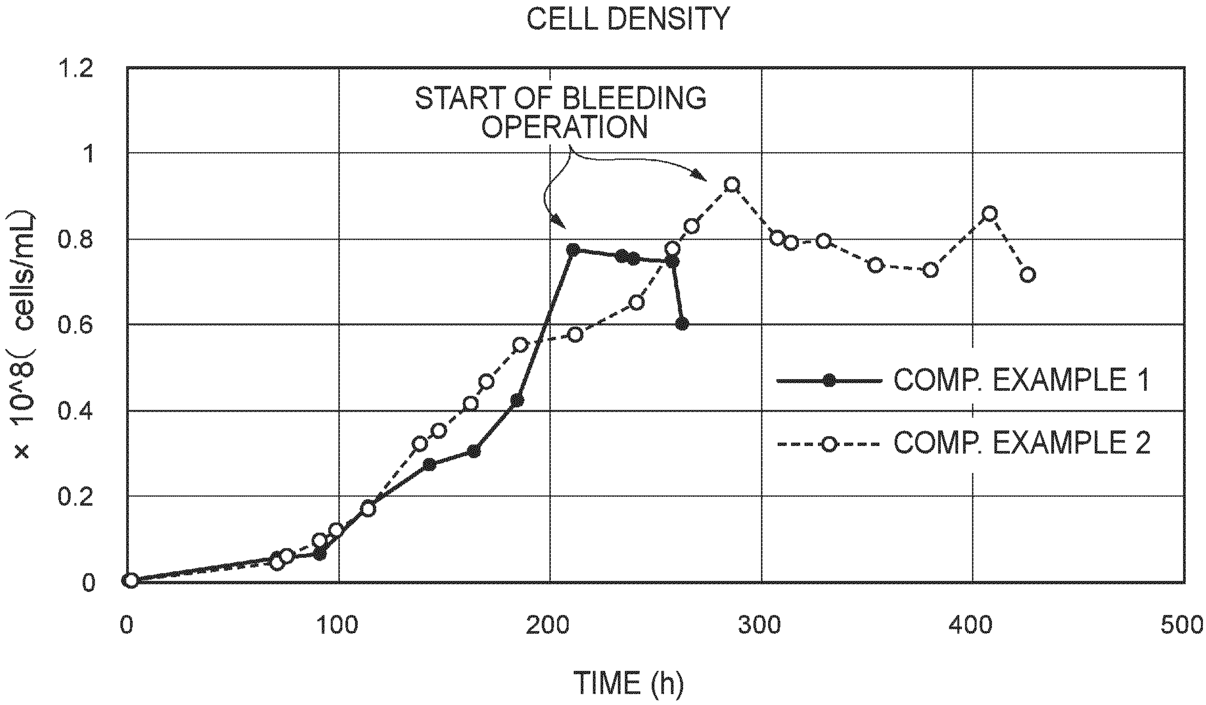
CELL DENSITY
1.2
1
0.8
0.6
0.4
0.2
0
× 10^8( cells/mL)
START OF BLEEDING OPERATION
COMP. EXAMPLE 1
COMP. EXAMPLE 2
0
100
200
300
400
500
TIME (h)

# Fig. 10

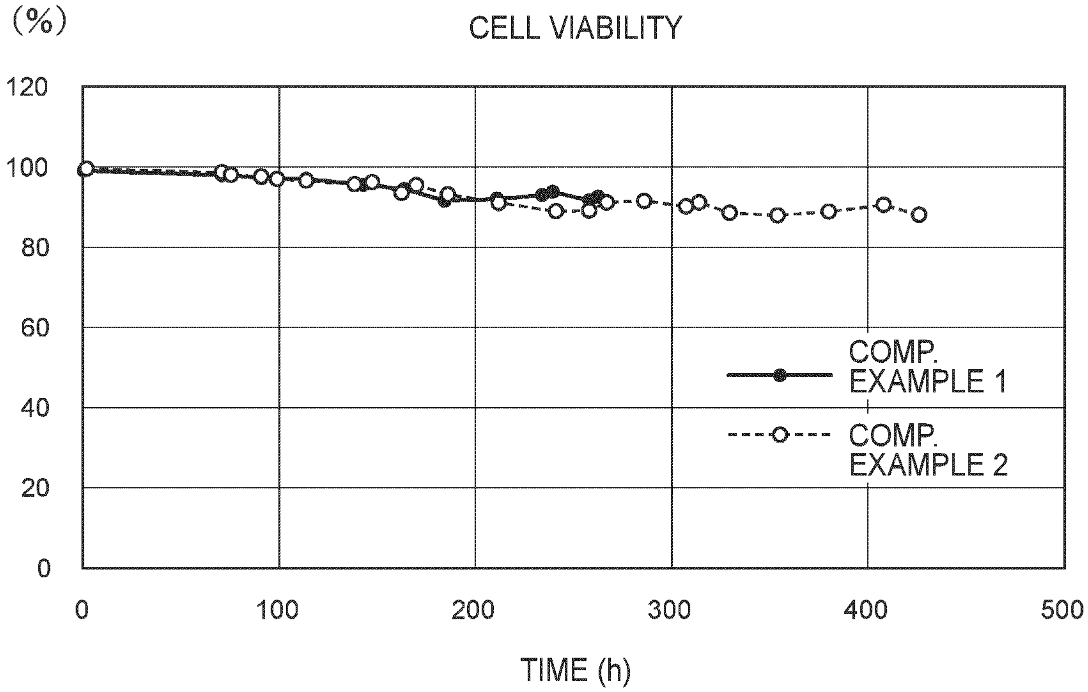
(%)
CELL VIABILITY
120
100
80
60
40
20
0
0
100
200
300
400
500
TIME (h)
COMP. EXAMPLE 1
COMP. EXAMPLE 2

# Fig. 11

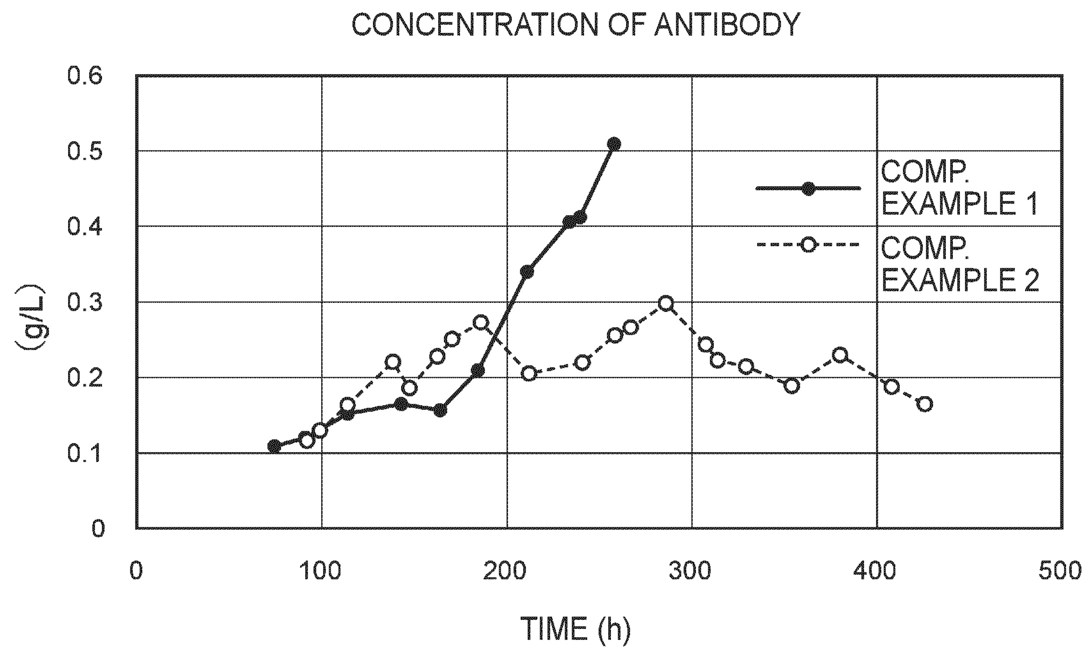
CONCENTRATION OF ANTIBODY
(g/L)
0.6
0.5
0.4
0.3
0.2
0.1
0
0
100
200
300
400
500
TIME (h)
COMP. EXAMPLE 1
COMP. EXAMPLE 2

Fig. 12

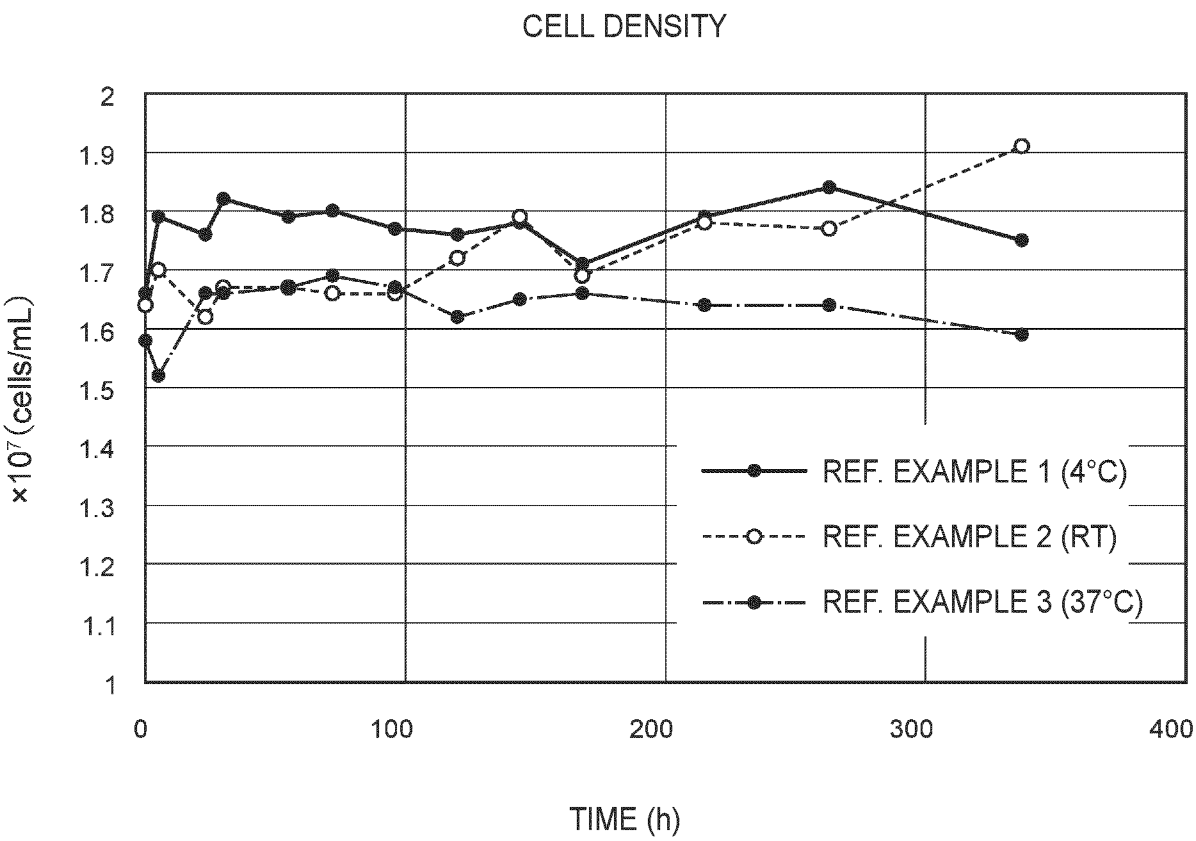
CELL DENSITY
2
1.9
1.8
1.7
1.6
1.5
1.4
1.3
1.2
1.1
1
×10^7 (cells/mL)
0
100
200
300
400
TIME (h)
REF. EXAMPLE 1 (4°C)
REF. EXAMPLE 2 (RT)
REF. EXAMPLE 3 (37°C)

Fig. 13

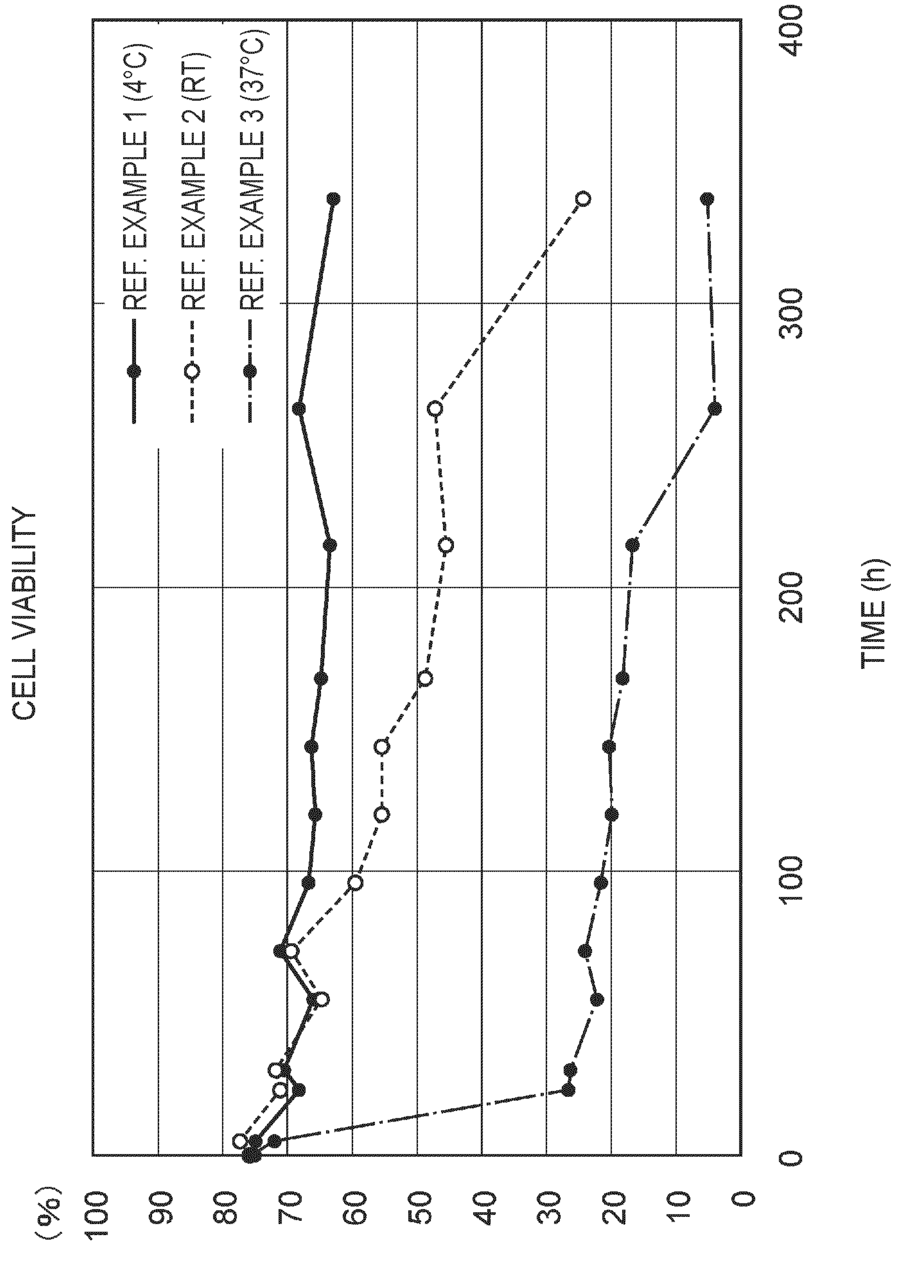
CELL VIABILITY
(%)
100
90
80
70
60
50
40
30
20
10
0
100
200
300
400
REF. EXAMPLE 1 (4°C)
REF. EXAMPLE 2 (RT)
REF. EXAMPLE 3 (37°C)
TIME (h)

Fig. 14

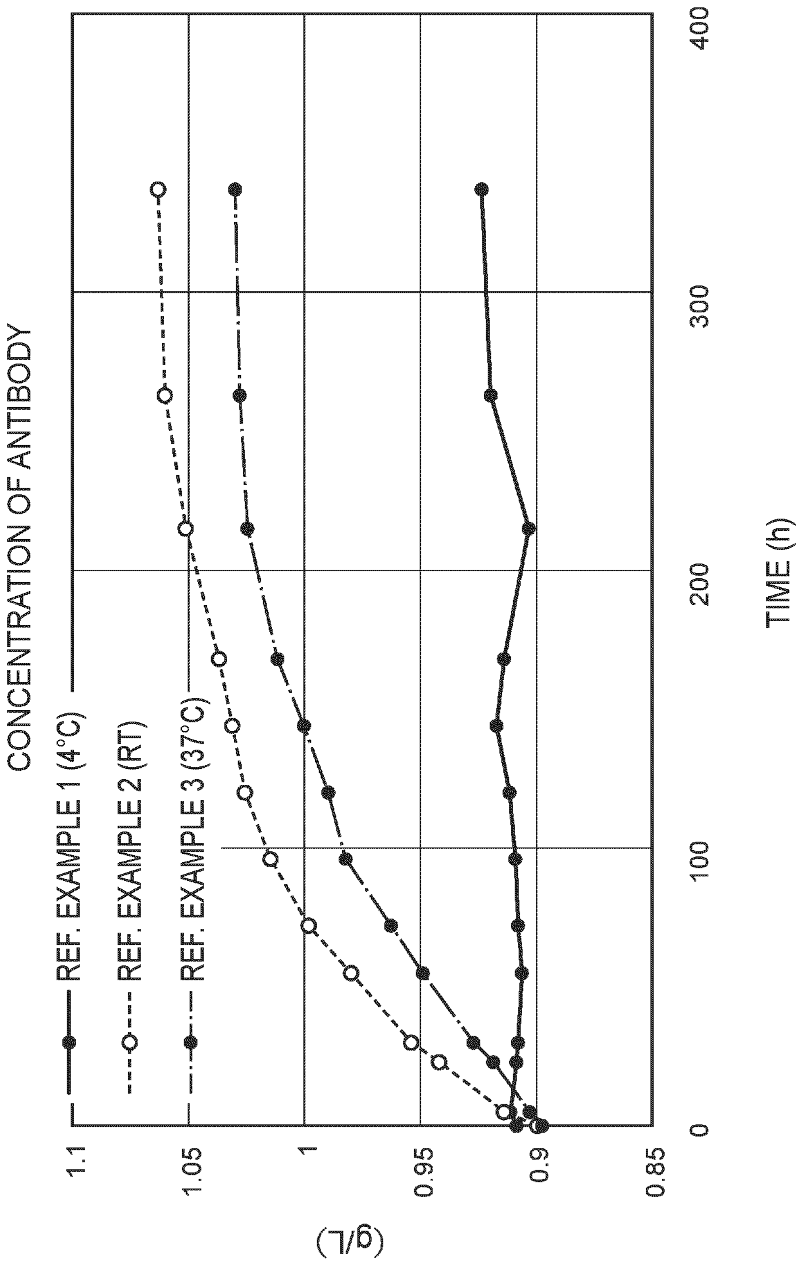
CONCENTRATION OF ANTIBODY
REF. EXAMPLE 1 (4°C)
REF. EXAMPLE 2 (RT)
REF. EXAMPLE 3 (37°C)
(g/L)
1.1
1.05
1
0.95
0.9
0.85
0
100
200
300
400
TIME (h)

Fig. 15

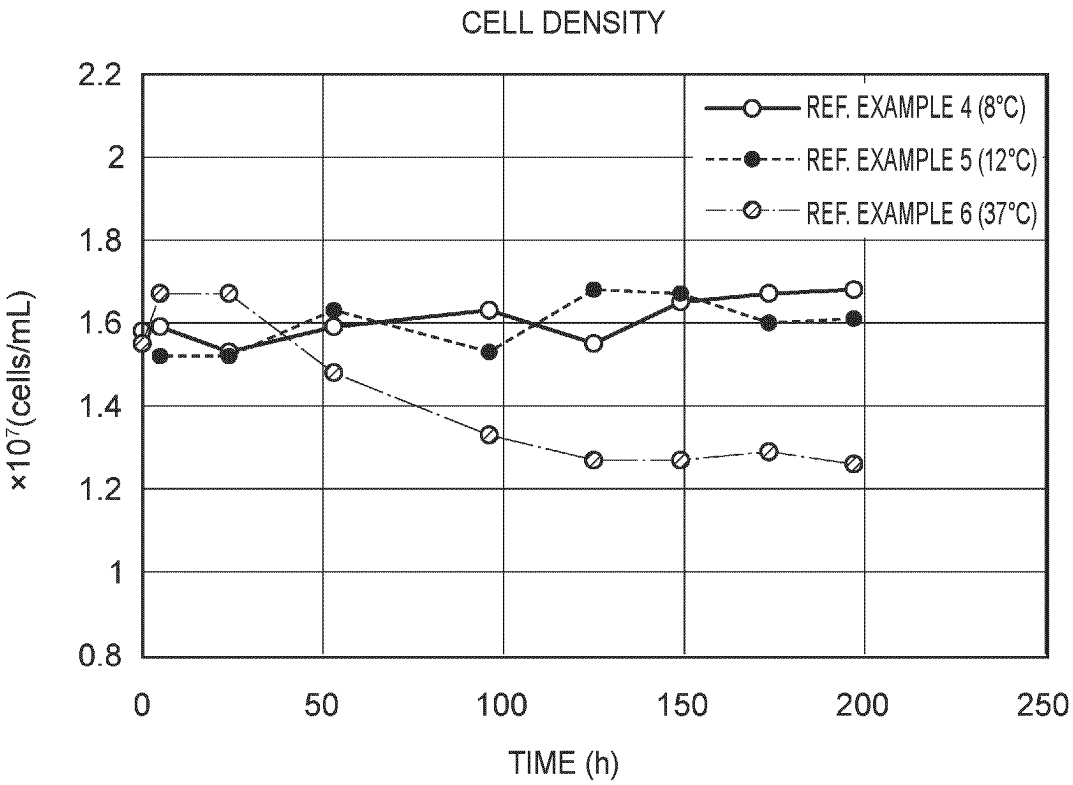
CELL DENSITY
2.2
2
1.8
1.6
1.4
1.2
1
0.8
×10⁷(cells/mL)
0
50
100
150
200
250
TIME (h)
REF. EXAMPLE 4 (8°C)
REF. EXAMPLE 5 (12°C)
REF. EXAMPLE 6 (37°C)

Fig. 16

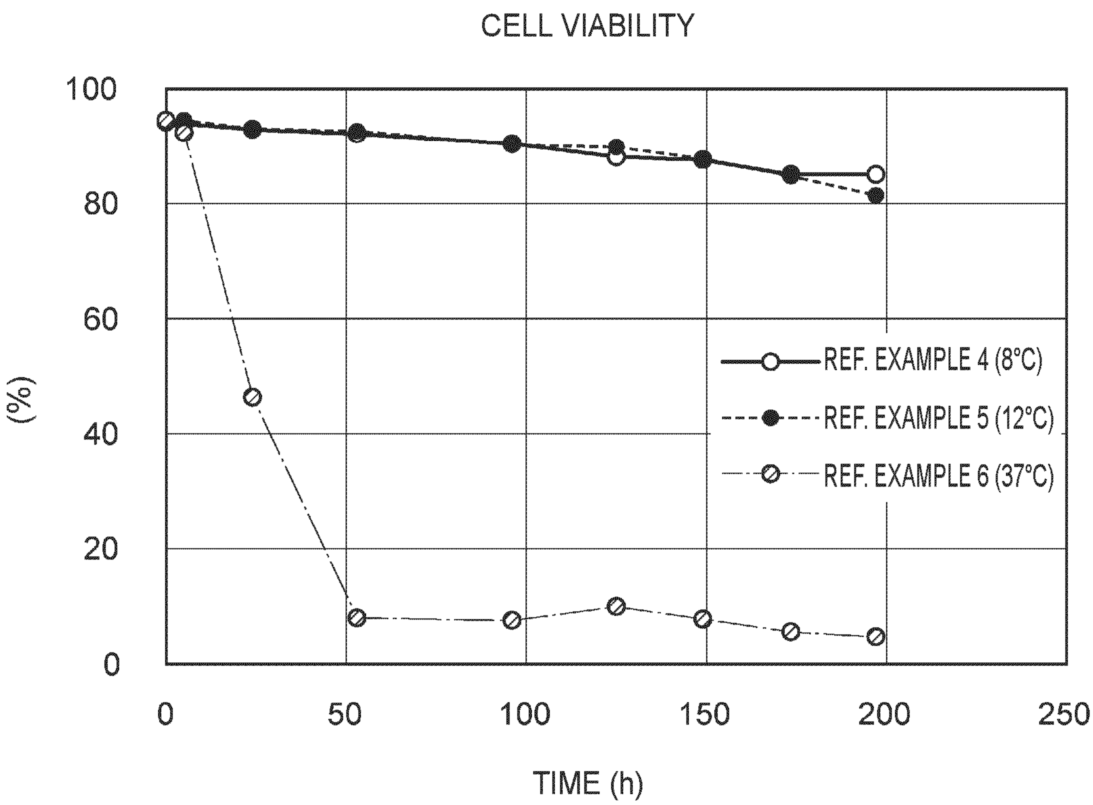
CELL VIABILITY
100
80
60
40
20
0
(%)
0
50
100
150
200
250
TIME (h)
REF. EXAMPLE 4 (8°C)
REF. EXAMPLE 5 (12°C)
REF. EXAMPLE 6 (37°C)

# Fig. 17

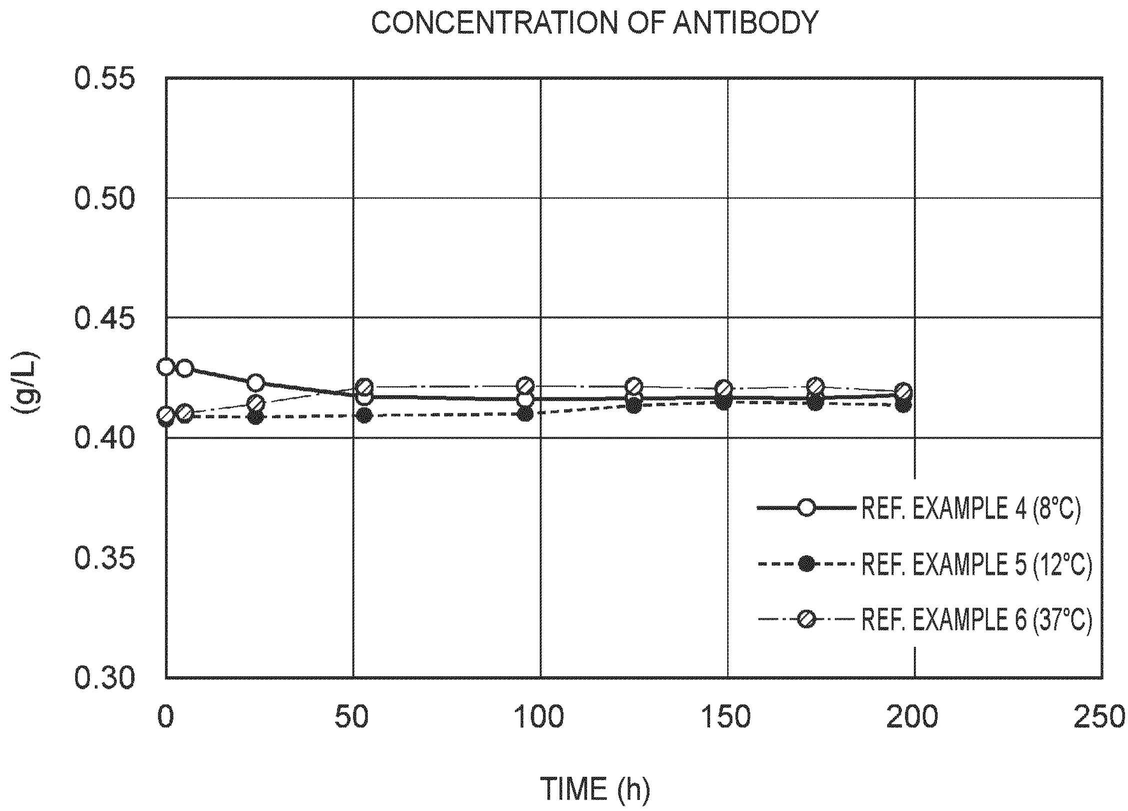
CONCENTRATION OF ANTIBODY
(g/L)
0.55
0.50
0.45
0.40
0.35
0.30
0
50
100
150
200
250
TIME (h)
REF. EXAMPLE 4 (8°C)
REF. EXAMPLE 5 (12°C)
REF. EXAMPLE 6 (37°C)

# Fig. 18

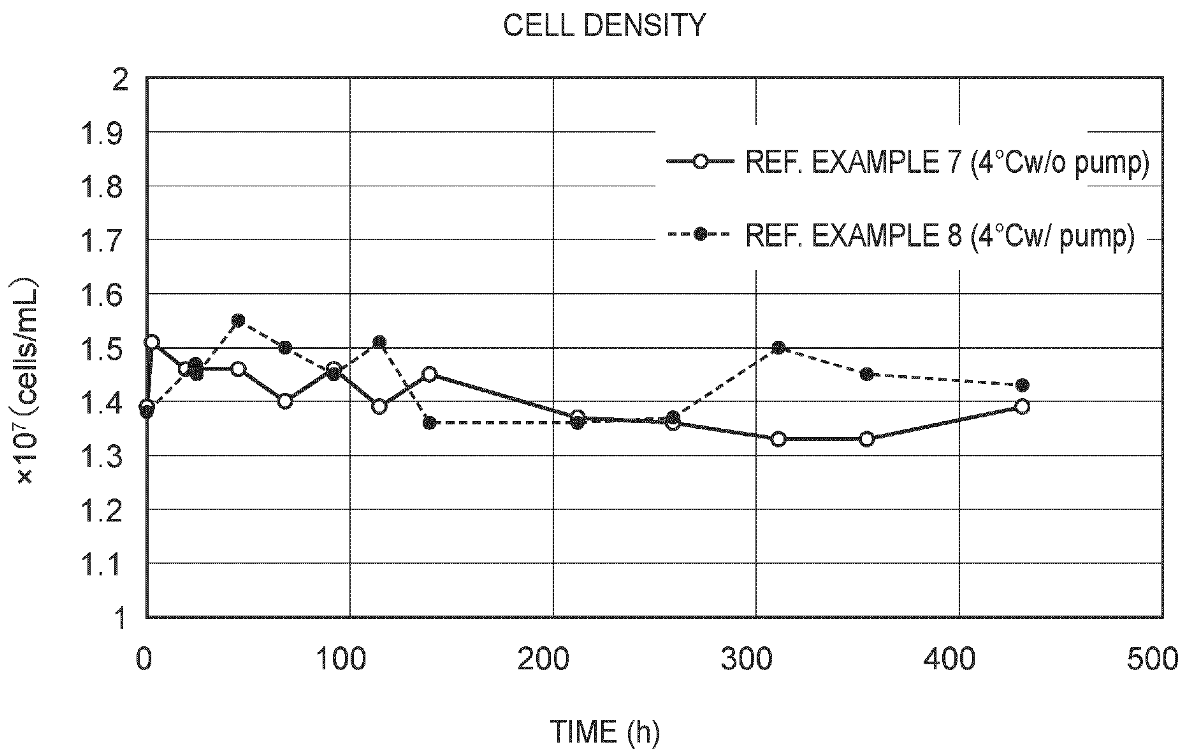
CELL DENSITY
×10⁷(cells/mL)
2
1.9
1.8
1.7
1.6
1.5
1.4
1.3
1.2
1.1
1
0
100
200
300
400
500
TIME (h)
REF. EXAMPLE 7 (4°Cw/o pump)
REF. EXAMPLE 8 (4°Cw/ pump)

Fig. 19

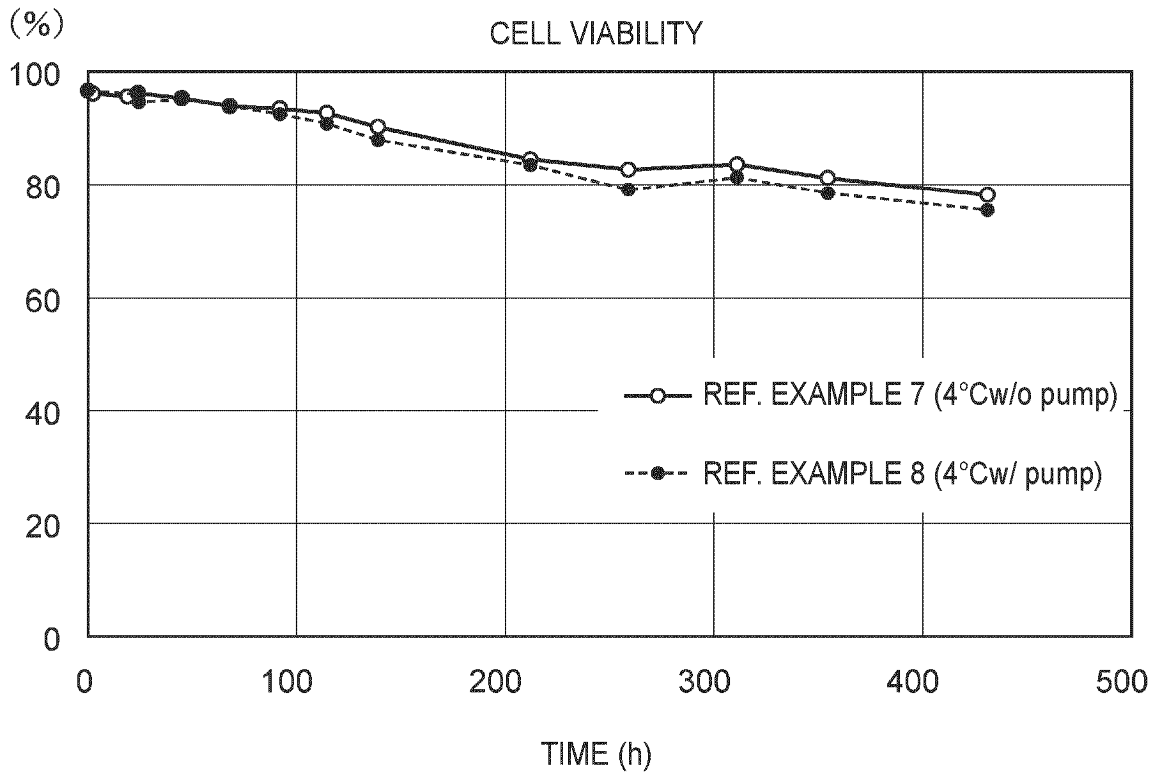
(%)
CELL VIABILITY
100
80
60
40
20
0
0
100
200
300
400
500
TIME (h)
REF. EXAMPLE 7 (4°Cw/o pump)
REF. EXAMPLE 8 (4°Cw/ pump)

Fig. 20

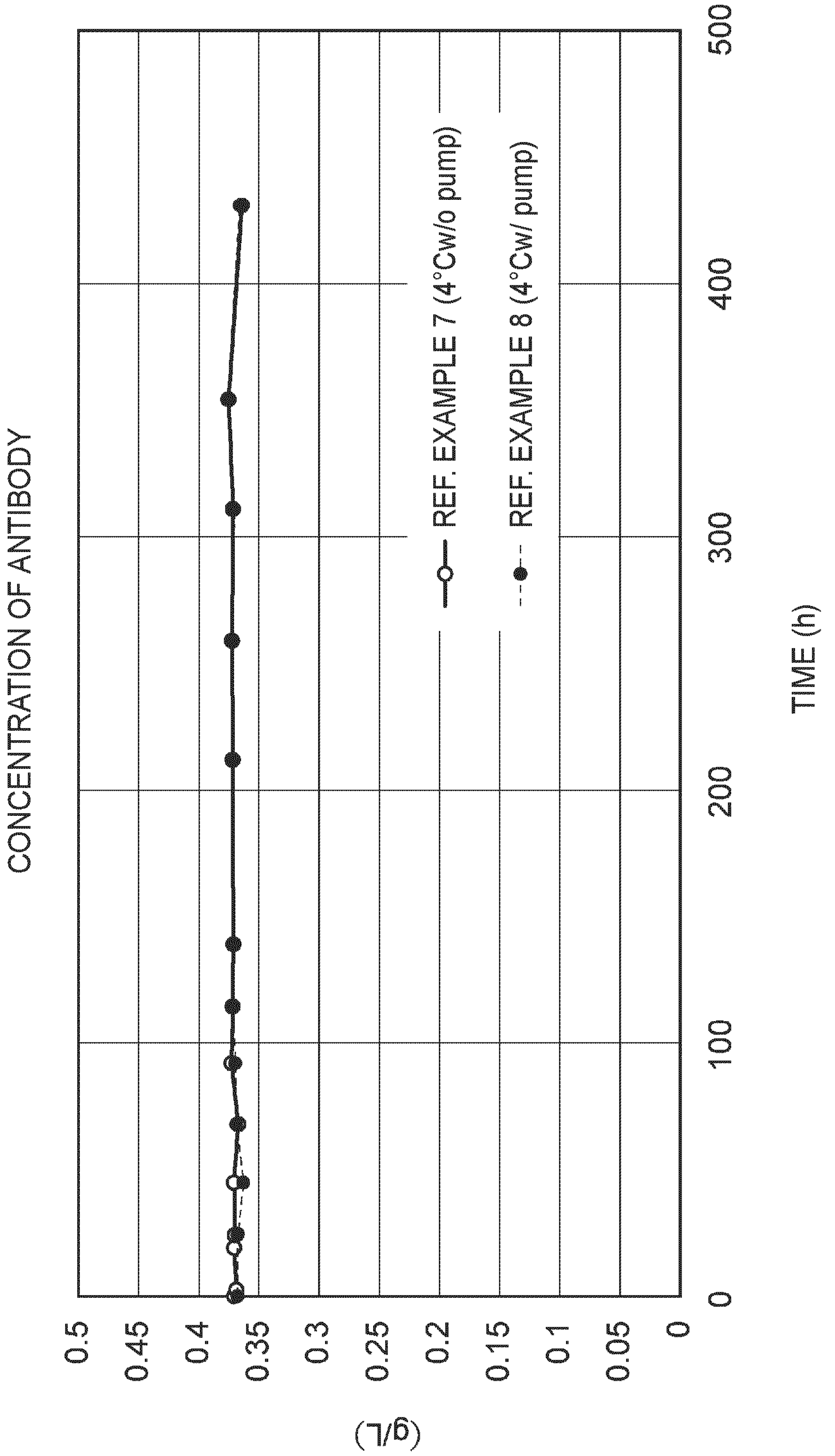
CONCENTRATION OF ANTIBODY
(g/L)
0.5
0.45
0.4
0.35
0.3
0.25
0.2
0.15
0.1
0.05
0
0
100
200
300
400
500
TIME (h)
REF. EXAMPLE 7 (4°Cw/o pump)
REF. EXAMPLE 8 (4°Cw/ pump)

## INTERNATIONAL SEARCH REPORT

International application No.
**PCT/JP2023/040996**

### A. CLASSIFICATION OF SUBJECT MATTER

***C12P 21/08***(2006.01)i; ***B01D 61/14***(2006.01)i; ***B01D 61/20***(2006.01)i; ***B01D 63/02***(2006.01)i; ***B01D 69/00***(2006.01)i; ***B01D 69/02***(2006.01)i; ***B01D 71/06***(2006.01)i; ***B01D 71/34***(2006.01)i; ***B01D 71/68***(2006.01)i; ***C12M 1/00***(2006.01)i; ***C12M 1/12***(2006.01)i; ***C12P 1/00***(2006.01)i; ***C12P 21/02***(2006.01)i; ***C12Q 1/02***(2006.01)i; ***C12Q 1/48***(2006.01)i
FI: C12P21/08; B01D61/14 500; B01D61/20; B01D63/02; B01D69/00; B01D69/02; B01D71/06; B01D71/34; B01D71/68; C12M1/00 A; C12M1/12; C12P1/00; C12P21/02 C; C12Q1/02; C12Q1/48 Z

According to International Patent Classification (IPC) or to both national classification and IPC

### B. FIELDS SEARCHED

Minimum documentation searched (classification system followed by classification symbols)

C12P21/08; B01D61/14; B01D61/20; B01D63/02; B01D69/00; B01D69/02; B01D71/06; B01D71/34; B01D71/68; C12M1/00; C12M1/12; C12P1/00; C12P21/02; C12Q1/02; C12Q1/48

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | WO 2010/038613 A1 (TORAY INDUSTRIES, INC) 08 April 2010 (2010-04-08)<br>claims, reference example 2, example 1 | 1-68 |
| A | US 2019/0169559 A1 (BOEHRINGER INGELHEIM INTERNATIONAL GMBH) 06 June 2019 (2019-06-06)<br>claims, examples | 1-68 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2024** | **06 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/040996**

| Patent document cited in search report | Publication date (day/month/year) | Patent family member(s) | Publication date (day/month/year) |
|---|---|---|---|
| WO 2010/038613 A1 | 08 April 2010 | US 2011/0177551 A1 claims, reference example 2, example 1 | |
| | | US 2016/0168601 A1 | |
| | | EP 2330209 A1 | |
| | | AU 2009299152 A1 | |
| | | CA 2738981 A1 | |
| | | CN 102171349 A | |
| | | RU 2011117359 A | |
| | | BR PI0913137 A2 | |
| | | KR 10-2011-0060888 A | |
| | | CN 106011187 A | |
| US 2019/0169559 A1 | 06 June 2019 | WO 2017/180814 A1 | |
| | | EP 3443064 A1 | |
| | | SG 11201809051Y A | |
| | | CN 109072154 A | |
| | | KR 10-2018-0134407 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018076291 A
- JP 2009045019 A
- JP 2021048776 A
- JP 5696479 B
- JP 2022516516 W